(19) Europäisches Patentamt
European Patent Office
Office européen des brevets

(11) Veröffentlichungsnummer: **0 217 372 B1**

(12) **EUROPÄISCHE PATENTSCHRIFT**

(45) Veröffentlichungstag der Patentschrift: **16.01.91**

(21) Anmeldenummer: **86113486.4**

(22) Anmeldetag: **01.10.86**

(51) Int. Cl.$^5$: **C 07 D 311/92,** C 07 D 405/12, C 07 D 473/08, A 61 K 31/35, A 61 K 31/395, A 61 K 31/02

(54) **Neue polyoxygenierte Labdan-Derivate, Verfahren zu ihrer Herstellung und ihre Anwendung als Medikamente.**

(30) Priorität: **02.10.85 DE 3535086**

(43) Veröffentlichungstag der Anmeldung:
**08.04.87 Patentblatt 87/15**

(45) Bekanntmachung des Hinweises auf die Patenterteilung:
**16.01.91 Patentblatt 91/03**

(84) Benannte Vertragsstaaten:
**AT BE CH DE ES FR GB GR IT LI LU NL SE**

(56) Entgegenhaltungen:
**EP-A-0 191 166**
**EP-A-0 193 132**
**EP-A-0 222 413**
**DE-A-2 654 796**
**US-A-4 639 443**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Khandelwal, Yatendra, Dr.**
**M-8, Hoechst Executive Quarters Darga Road**
**Mulund (West) Bombay 400 082 (IN)**
Erfinder: **Rajgopalan, Ramanujam No. 205, B Wing**
**Aarthi Sarojini Naidu Road**
**Mulund (West) Bombay 400 080 (IN)**
Erfinder: **Dohadwalla, Alihussein Nomanbhai, Dr.**
**Noman Masion, 139 C**
**Cumballa Hill Bombay 400 036 (IN)**
Erfinder: **Rupp, Richard Helmut, Dr.**
**"Niladri" 66 Nepean Sea Road**
**Bombay 400 006 (IN)**
Erfinder: **DE Souza, Noel John, Dr.**
**Melrose 62 Pali Hill**
**Bandra Bombay 400 050 (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 217 372 B1

**Beschreibung**

Polyoxygenierte Labdan-Derivate sind bereits aus den folgenden Patentschriften bzw. Publikationen bekannt:

Deutsche Offenlegungsschriften Nr. 25 57 784, 26 40 275 und 26 54 796; Europäische Patentanmeldungen Veröffentlichungsnummer 0 189 801, 0 191 166 und 0 193 132, Tetrahedron Letters Nr. 19, S. 1669—1672 (1977), J. Chem. Soc., Perkin Trans. 1, 767 (1982).

Die pharmakologischen Eigenschaften der beschriebenen polyoxygenierten Labdane und ihrer Derivate, machen sie für die Anwendung bei der Behandlung von Herz- und Kreislauferkrankungen, Hypertonie, Glaukom, Allergie, Bronchokonstriktion und als Immunomodulatoren geeignet.

Die erfindungsgemäßen polyoxygenierten Labdan-Derivate sind in der bisher bekannt gewordenen Literatur noch nicht beschrieben worden. Die Verbindungen gemäß dem genannten Stand der Technik, die strukturell mit den erfindungsgemäßen Verbindungen verwandt sind, sind Labdan-Derivate, in denen eine oder mehrere Hydroxyl-Gruppen acyliert sind. Im Gegensatz dazu bestizen die erfindungsgemäßen Verbindungen eine Aminoacyl-Gruppe. Dadurch werden die erfindungsgemäßen Verbindungen wasserlöslich, eine Eigenschaft, welche den Verbindungen gemäß dem Stand der Technik fehlt. Dank dieser Wasserlöslichkeit können die erfindungsgemäßen Verbindungen z.B. sehr günstig in wäßrigen galenischen Zubereitung z.B. in Injektionslösungen verwendet werden. Ein weiterer Vorteil der Einführung von Aminoacyl-Gruppen besteht darin, daß je nach struktureller Beschaffenheit eines solchen Substituenten und seiner Stellung im Molekül das pharmakologische Profil der Verbindungen so verändert wird, daß es im Unterschied zu den bereits bekannten Verbindungen möglich wird, Substanzen zu erhalten, die beispielsweise in einem Falle bei der Behandlung von Erkrankungen wie z.B. der konstriktiven Kardiomyophatie oder in einem anderen Falle bei Bluthochdruck oder schließlich bei Erkrankungen wie dem Glaukom besser wirksam sind.

Die vorliegende Erfindung betrifft daher Derivate von polyoxygenierten Labdanen und Verfahren zu ihrer Herstellung. Die neuen polyoxygenierten Labdan-Derivate und deren pharmazeutisch verwendbare Salze besitzen wertvolle Eigenschaften wie beispielsweise broncho-spasmolytische, antiallergische, blutdrucksenkende, antiphlogistische und positivinotrope Wirkung. Sie bewirken ferner die Senkung des Augeninnendruckes beim Glaukom.

Die neuen Derivate der polyoxygenierten Labdane besitzen die Formel I,

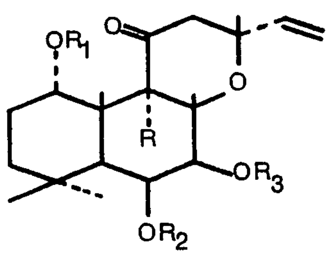

I

worin debeuten:

R Wasserstoff oder Hydroxyl;

$R_2$ den Rest

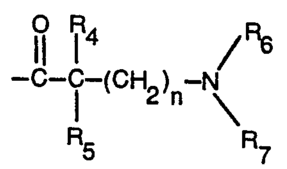

und

$R_1$ und $R_3$ Wasserstoff,

wobei

$R_4$ und $R_5$ gleich oder verschieden sind und Wasserstoff oder $C_1$—$C_4$-Alkyl darstellen,

n für 0 oder eine ganze Zahl von 1 bis 4 steht,

$R_6$ Wasserstoff bedeutet und

$R_7$ Wasserstoff, unsubstituiertes oder substituiertes $C_1$—$C_4$-Alkyl, Cyclohexyl, Phenyl oder Phenyl-$C_1$—$C_2$-alkyl, wobei der Phenylrest ein- oder mehrfach mit Halogen, $C_1$—$C_4$-Alkyl, $C_1$—$C_4$-Alkoxy, Nitro und/oder Trifluormethyl substituiert sein kann, Pyridin, Chinolin, gegebenenfalls substituiertes Amino, Hydroxy, $C_1$—$C_6$-Alkanoyl, $C_2$—$C_6$-Alkenoyl, Benzoyl oder Phenyl-$C_1$—$C_6$-alkanoyl, worin der Phenylrest ein- oder mehrfach mit Halogen, $C_1$—$C_4$-Alkyl, $C_1$—$C_4$-Alkoxy, Nitro oder Trifluormethyl substituiert sein kann, Di-$C_1$—$C_4$-alkylamino, Carbamyl, $C_1$—$C_4$-Alkoxycarbonyl, $C_1$—$C_4$-Alkoxycarbonyl-$C_1$—$C_4$-alkyl, oder

$R_6$ und $R_7$ die gleiche Bedeutung haben und gegebenenfalls substituiertes $C_1$—$C_4$-Alkyl oder Phenyl oder Phenyl-$C_1$—$C_2$-Alkyl darstellen, wobei die Phenylreste wie oben angegeben substituiert sein können, oder

$R_6$ $C_1$—$C_4$-Alkyl bedeutet und $R_7$ substituiertes $C_1$—$C_4$-Alkyl oder Cyclohexyl, Phenyl-$C_1$—$C_4$-alkyl,

worin der Phenylrest ein- oder mehrfach mit Halogen, $C_1$—$C_4$-Alkyl, $C_1$—$C_4$-Alkoxy, Nitro und/oder Trifluormethyl substituiert sein kann, oder Di-$C_1$—$C_4$-alkylamino-$C_1$—$C_4$-alkyl darstellt, oder

$R_6$ und $R_7$ zusammen mit dem N-Atom, an das sie gebunden sind, den Piperidin-, Pyrrolidin-, Morpholin-, Piperazin-, Thiomorpholin-, Imidazol- oder Theophyllin-Rest darstellen, wobei die genannten Reste ein- oder mehrfach mit $C_1$—$C_4$-Alkyl, $C_1$—$C_4$-Alkoxy, Phenyl, Phenyl-$C_1$—$C_2$-alkyl, Hydroxy, Amino- oder Hydroxy-$C_1$—$C_4$-alkyl oder substituierte Phenyl- oder Aminogruppen substituiert sein können, sowie deren pharmakologisch unbedenklichen Salze.

Bevorzugt sind Verbindungen der Formel I, worin R Hydroxy ist und $R_2$ den Rest

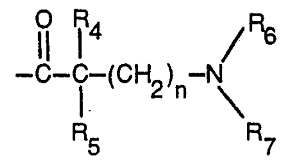

bedeutet, worin $R_4$ und $R_5$ Wasserstoff oder $R_4$ Wasserstoff und $R_5$ $C_1$—$C_4$-Alkyl, $R_6$ $C_1$—$C_4$-Alkyl und $R_7$ Cyclohexyl, oder $R_6$ und $R_7$ zusammen mit dem N-Atom, an das sie gebunden sind, den Piperidino-, Morpholino-, Piperazino- oder Pyrrolidino-Rest darstellen, und n 0 ist, sowie ihre pharmakologisch unbedenklichen Säureadditionssalze.

Die vorstehende Definition der Derivate polyoxygenierter Labdane umfaßt sämtliche möglichen Stereoisomere und deren Kombinationen.

Geeignete Beispiele der Alkylgruppen für $R_4$ bis $R_7$ sind gerade oder verzweigte Alkylgruppen mit bis zu 4 Kohlenstoffatom, beispielsweise der Methyl-, Äthyl-, Isopropyl-, t-Butyl- oder n-Butyl-Rest.

Geeignete Beispiele der substituierten $C_1$—$C_4$-Alkyl-Gruppen für $R_6$ und $R_7$ sind Hydroxy-$C_1$—$C_4$-Alkyl wie beispielsweise Hydroxy-Äthyl, Carboxy-$C_1$—$C_4$-Alkyl wie beispielsweise Carboxy-Äthyl, Carbalkoxy-$C_1$—$C_4$-Alkyl wie beispielsweise Carbäthoxy-Äthyl oder die Äthyl-Indol-Gruppe.

Ein geeignetes Beispiel für $R_6$ und $R_7$ in Bedeutung einer Phenylalkyl-Gruppe ist z.B. die Benzylgruppe.

Beispiele für $C_1$—$C_6$-Alkanoyl-Gruppen sind Formyl, Acetyl, Propionyl, Butyryl, Isobutyryl, Valeryl und Bromisobutyryl. Die Alkanoyl-Gruppen können eine Doppelbindung wie beispielsweise in der Acryloyl-Gruppe enthalten.

Ein Beispiel für Phenyl-$C_1$—$C_6$-alkanoyl ist die Phenylacetyl-Gruppe.

Geeignet als Di-$C_1$—$C_4$-alkylamino-$C_1$—$C_4$-alkyl-Gruppe für $R_7$ ist z.B. die Diäthyl-aminoäthyl-Gruppe.

Geeignete Beispiele für Salze der Substanzen der Erfindung aus anorganischen und organischen Säuren sind Hydrochlorid, Hydrobromid, Sulfat, Phosphat, Acetat, Oxalat, Tartarat, Zitrat, Maleat oder Fumarat.

Geeignete Beispiele für die Gruppe

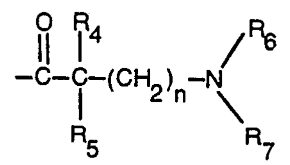

sind die folgenden Reste:

N-Äthylaminoacetyl
N,N-Diäthylaminoacetyl
N-Benzyl-N-Methylaminoacetyl
N-Äthyl-N-Methylaminoacetyl
4'-Phenyl-Piperidinoacetyl
Anilinacetyl

N,N-Diphenylaminoacetyl
β-Phenyläthylaminoacetyl
N-Acetylaminoacetyl
N-Phenylacylaminoacetyl
4'-Hydroxypiperidinoacetyl
Thiomorpholinoacetyl
Isopropylaminoacetyl
t-Butylaminoacetyl
Diäthanolaminoacetyl
N(N',N'-Diäthylaminoäthyl),N-Methylaminoacetyl
Hydrazinacetyl
Hydroxyaminoacetyl
Phenylhydrazinacetyl
Aminoacetyl
Benzylaminoacetyl

4-Benzoylaminopiperidinoacetyl
4-Hydroxy-4-phenylpiperidinoacetyl
β-Alaninacetyl
t-Butyl-β-Alaninacetyl
Tryptaminacetyl
Aminochinolinacetyl
Imidazolylacetyl
Theophyllinacetyl
β-(N-Äthylamino)-propionyl
β-(N,N-Diäthylamino)-propionyl
β-(N-Benzyl-N-methylamino)-propionyl
β-(4′-Phenylpiperidin)-propionyl
β-(Anilin)-propionyl

β-(N,N-Diphenylamino)-propionyl
β-(β′-Phenyläthylamino)-propionyl
β-(N-Acetylamino)-propionyl
β-(N-Phenacylamino)-propionyl
β-(4′-Hydroxypiperidin)-propionyl
β-(Thiomorpholin)-propionyl
β-(Isopropylamino)-propionyl
β-(t-Butylamino)-propionyl
β-(Diäthanolamino)-propionyl
β-N-(N′,N′-Diäthylamino)-Äthyl,N-methylamino-propionyl
β-Hydrazinpropionyl
β-Hydroxylamino-propionyl
β-Penylhydrazin-propionyl
β-Aminopropionyl
β-Benzylamin-propionyl

β-(4-Benzoylaminopiperidin)-propionyl
β-(4-Hydroxy-4-phenylpiperidin)-propionyl
β-(β′-Alanin)-propionyl
β-(t-Butyl-β′-alanin)-propionyl
β-Tryptamin-propionyl
β-Aminochinolin-propionyl
β-Imidazolyl-propionyl
β-Theophyllin)-propionyl
α-(N-Äthylamino)-propionyl
α-(N,N-Diäthylamino)-propionyl
α-(N-Benzyl-N-methylamino)-propionyl
α-(N-Äthyl-N-methylamino)-propionyl
α-(4′-Phenylpiperidin)-propionyl
α-(Anilin)-propionyl

α-(N,N-Diphenylamino)-propionyl
α-(β′-Phenyläthylamino)-propionyl
α-(N-Acetylamino)-propionyl
α-(Phenacylamino)-propionyl
α-(4′-Hydroxypiperidin)-propionyl
α-(Thiomorpholin)-propionyl
α-(Isopropylamino)-propionyl
α-(t-Butylamino)-propionyl
α-(Diäthanolamino)-propionyl
α-(N,N′-Diäthylamino)-Äthyl,N-Methylamino-propionyl
α-(Hydrazin)-propionyl
α-(Hydroxyamin)-propionyl
α-(Phenylhydrazin)-propionyl
α-(Amino)-propionyl
α-(Benzylamin)-propionyl

α-(4-Benzoylamino-piperidin)-propionyl
α-(4-Hydroxy-4-phenylpiperidin)-propionyl
α-(β-Alanin)-propionyl
α-(t-Butylalanin)-propionyl

α-(Tryptamin)-propionyl
α-(Aminochinolin)-propionyl
α-Imidazolyl-propionyl
α-Theophyllin-propionyl
γ-(N-Äthylamino)-butyryl
γ-(N,N-Diäthylamino)-butyryl
γ-(N-Benzyl-N-methylamino)-butyryl
γ-(N-Äthyl-N-methylamino)-butyryl
γ-(4'-Phenylpiperidin)-butyryl
γ-Anilin-butyryl

γ-(N,N-Diphenylamino)-butyryl
γ-(β'-Phenyl-Äthylamino)-butyryl
γ-(N-Acetylamino)-butyryl
γ-(N-Phenacylamino)-butyryl
γ-(4'-Hydroxypiperidino)-butyryl
γ-(Thiomorpholino)-butyryl
γ-(Isopropylamino)-butyryl
γ-(t-Butylamino)-butyryl
γ-(Diäthanolamino)-butyryl
γ-(N(N',N'-Diäthylamino)-äthyl,N-methylamino)-butyryl
γ-Hydrazino-butyryl
γ-Hydroxyamino-butyryl
γ-Phenylhydrazino-butyryl
γ-(Amino)-butyryl
γ-(Benzylamino)-butyryl

γ-(4-Benzoylamino-piperidino)-butyryl
γ-(4-Hydroxy-4-phenylpiperidino)-butyryl
γ-(β'Alanino)-butyryl
γ-(t-Butyl-β'-Alanino)-butyryl
γ-(Tryptamino)-butyryl
γ-(Aminochinolino)-butyryl
γ-Imidazolyl-butyryl
δ-(Theophyllino)-butyryl
δ-(N-Äthylamino)-valeryl
δ-(N,N-Diäthylamino)-valeryl
δ-(N-Benzyl-N-methylamino)-valeryl
δ-(N-Äthyl-N-methylamino)-valeryl
δ-(4'-Phenylpiperidino)-valeryl
δ-(Anilino)-valeryl

δ-(N,N-Diphenylamino)-valeryl
δ-(β-Phenyläthylamino)-valeryl
δ-(N-Acetylamino)-valeryl
δ-(N-Phenacylamino)-valeryl
δ-(4'-Hydroxypiperidino)-valeryl
δ-(Thiomorpholino)-valeryl
δ-(Isopropylamino)-valeryl
δ-(t-Butylamino)-valeryl
δ-(Diäthanolamino)-valeryl
δ-(N(N',N'-Diäthylamino)-äthyl-N-methylamino)-valeryl
δ-(Hydrazino)-valeryl
δ-(Amino)-valeryl
δ-(Benzylamino)-valeryl
δ-(Phenylhydrazino)-valeryl

δ-(4-Benzoylaminpiperidino)-valeryl
δ-(4-Hydroxy-4-phenylpiperidino)-valeryl
δ-(β-Alanino)-valeryl
δ-(t-Butyl-β-Alanino)-valeryl
δ-(Tryptamino)-valeryl
δ-(Aminochinolin)-valeryl
δ-(Imidazolyl-valeryl
δ-Theophyllino-valeryl

Ohne ausschließlich auf die genannten Verbindungen beschränkt zu bleiben, sind die bevorzugten Verbindungen der Erfindung die folgenden:

8,13-Epoxy-6β-piperidinacetoxy-1α,7β,9α-trihydroxy-labd-14-en-11-on.

8,13-Epoxy-6β-morpholinoacetoxy-1α,7β,9α-trihydroxy-labd-14-en-11-on.

8,13-Epoxy-6β-(N-methylpiperazinoacetoxy-1α,7β,9α-trihydroxy-labd-14-en-11-on-dihydrochlorid.

8,13-Epoxy-6β-pyrrolidonoacetoxy-1α,7β,9α-trihydroxy-labd-14-en-11-on.

8,13-Epoxy-6β-(N-methyl-N-cyclohexylaminoacetoxy-1α,7β,9α-trihydroxy-labd-14-en-11-on.

8,13-Epoxy-6β-(α'(N-morpholino)propionyloxy)-1α,7β,9α-trihydroxy-labd-14-en-11-on-hydrochlorid.

8,13-Epoxy-6β-(α'(4N-methylpiperazino)propionyloxy)-1α,7β,9α-trihydroxy-labd-14-en-11-on.

8,13-Epoxy-6β-(α'(N-piperidino)propionyloxy)-1α,7β,9α-trihydroxy-labd-14-en-11-on-hydrochlorid.

1α,7β-Dihydroxy-8,13-epoxy-6β-(piperidinoacetoxy)-labd-14-en-11-on.

8,13-Epoxy-6β-(4'-hydroxypiperidinoacetoxy)-1α,7β,9α-trihydroxy-labd-14-en-11-on-hydrochlorid-dihydrat.

8,13-Epoxy-6β-(thiomorpholinoacetoxy)-1α,7β,9α-trihydroxy-labd-14-en-11-on-hydrochlorid-sesquihydrat.

8,13-Epoxy-6β-(4'-methylpiperidinoacetoxy)-1α,7β,9α-trihydroxy-labd-14-en-11-on-hydrochlorid-dihydrat.

8,13-Epoxy-6β(γ'-piperidinobutyryloxy)-1α,7β,9α-trihydroxy-labd-14-en-11-on-hydrochlorid-hemihydrat.

8,13-Epoxy-6β(diethylaminoacetoxy)-1α,7β,9α-trihydroxy-labd-14-en-11-on-monohydrochlorid-dihydrat.

8,13-Epoxy-6β(γ'-morpholinobutyryloxy)-1α,7β,9α-trihydroxy-labd-14-en-11-on-hydrochlorid-dihydrat.

8,13-Epoxy-6β(α'-anilinopropionyloxy)-1α,7β,9α-trihydroxy-labd-14-en-11-on.

8,13-Epoxy-6β((4'-hydroxy-4'-phenylpiperidinoacetoxy)-1α,7β,9α-trihydroxy-labd-14-en-11-on-hydrat.

8,13-Epoxy-6β(theophyllinoacetoxy)-1α,7β,9α-trihydroxy-labd-14-en-11-on.

8,13-Epoxy-6β(benzylaminoacetoxy)-1α,7β,9α-trihydroxy-labd-14-en-11-on-hydrochlorid-monohydrat.

8,13-Epoxy-6β(3'-methylpiperidino-acetoxy)-1α,7β,9α-trihydroxy-labd-14-en-11-on-hydrochloridsesquihydrat.

8,13-Epoxy-6β(δ'-piperidinovaleryloxy)-1α,7β,9α-trihydroxy-labd-14-en-11-on-monohydrochlorid-monohydrat. Fp. 148—150°C.

Gegenstand der Erfindung ist ferner ein Verfahren zur Herstellung der neuen, polyoxygenierten Labdan-Derivate der Formel I und ihrer pharmazeutisch unbedenklichen Salze. Ausgangsmaterialien für die Herstellung dieser Derivate sind zwei Verbindungen, dargestellt durch die Formel II,

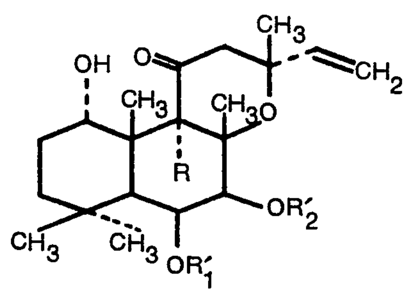

II

in der R für Wasserstoff oder die Hydroxylgruppe steht und $R'_1$ und $R'_2$ Wasserstoff sind. Die Verbindung der Formel II, bei der R OH und $R'_1$ und $R'_2$ Wasserstoff bedeuten, ist ein Inhaltsstoff der Pflanze *Coleus forskohlii* und wird außerdem aus 6-Acetyl-7-Deacetylforskolin (II, R= OH, $R'_1$= Ac, $R'_2$= OH) durch Hydrolyse gewonnen. Ein weiterer Inhaltsstoff dieser Pflanze mit der Bezeichnung 9-Desoxyforskolin (II, R= H, $R'_1$= Ac) liefert bei der Hydrolyse die andere Ausgangssubstanz der Formel II, worin R und $R'_1$ für Wasserstoff stehen. Über die Gewinnung dieser Stoffe aus der Pflanze *Coleus forskohlii* wird in den Veröffentlichungen berichtet, die als Stand der Technik aufgeführt sind.

Das Verfahren zur Herstellung polyoxygenierten Labdan-Derivate der Formel I, in der R entweder für Wasserstoff oder eine Hydroxylgruppe, $R_1$ und $R_3$ für Wasserstoff und $R_2$ für die Gruppe

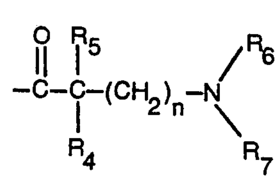

stehen und $R_4$, $R_5$, $R_6$, $R_7$ und n der vorstehend genannten Definition entsprechen, beinhaltet die Reaktion von Verbindungen der Formel II (R= H oder OH, $R'_1$=$R'_2$=H) mit einem Haloalkanoylhalid der Formel III,

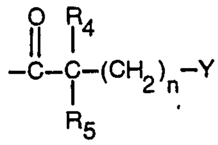

III

in der $R_4$, $R_5$ und n die gleiche Bedeutung haben wie für $R_4$, $R_5$ und n in Formel I definiert und X und Y für ein Halogenatom stehen in Gegenwart einer Base. Bei der verwendeten Base kann es sich um eine organische stickstoffhaltige Verbindung wie beispielsweise Pyridin oder Triäthylamin handeln. Die Reaktion kann bei Temperaturen zwischen 0°C und Zimmertemperatur und in Lösungsmitteln wie aromatischen Kohlenwasserstoff, beispielsweise Benzol, Toluol oder Äthern wie Tetrahydrofuran oder Dioxan oder halogenierten Kohlenwasserstoffen wie Methylenchlorid oder Chloroform durchgeführt werden. Die Reaktionsdauer beträgt eine halbe bis zu sechs Stunden. Das Produkt wird durch Eindampfen des Reaktionsgemisches, anschließende Extraktion mit einem organischen Lösungsmittel und Waschen des Extraktes nacheinander mit verdünnter Salzsäure, Wasser, Natriumbicarbonat-Lösung und Wasser, anschließendem Trocknen über Trocknungsmitteln wie wasserfreiem Natriumsulfat und schließlich durch Eindampfen im Vakuum isoliert. Bei dem verbliebenen Rückstand handelt es sich um eine Mischung von Verbindungen der Formel IV

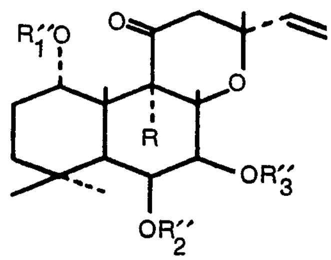

worin $R''_1$, $R''_2$ und $R''_3$ Wasserstoff oder den Rest

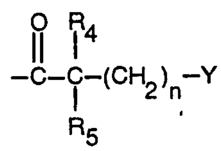

bedeuten, wobei mindestens einer der Substituenten $R''_1$ bis $R''_3$ den genannten Rest darstellt, die gegebenenfalls ohne Reinigung zur weiteren Reaktion mit Aminen der Formel V

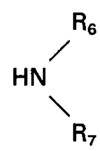

V

verwendet wird, in der $R_6$ und $R_7$ die gleichen Bedeutungen haben wie sie für $R_6$ und $R_7$ in Formel I definiert worden sind. Die Reaktion erfolgt bei Temperaturen zwischen Raumtemperatur und 150°C über einen Zeitraum von 1 bis 6 Stunden mit oder ohne Lösungsmittel. Als Lösungsmittel dienen aromatische Kohlenwasserstoffe wie Benzol, Toluol oder Äther wie Tetrahydrofuran oder Dioxan. Das Produkt der Formel I wird aus dem Reaktionsgemisch mittels Konzentration des Reaktionsgemisches, anschließender Extraktion mit einem organischen Lösungsmittel wie Äthylacetat und durch Reinigung des Extraktes mittels Chromatographie über Silikagel und Eluieren mit Äthylacetat/Petroläther isoliert.

Verbindungen der Formel I, worin R Wasserstoff oder Hydroxy, $R_1$ Wasserstoff, $R_2$ den Rest

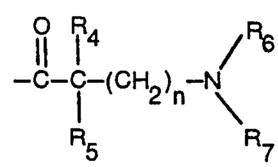

und $R_3$ Wasserstoff bedeutet, worin $R_4$ bis $R_7$ und n die zur Formel I genannten Bedeutungen haben, können durch Umsetzung einer Verbindung der Formel I, in der R und $R_1$ die genannte Bedeutung haben, $R_2$ Wasserstoff und $R_3$ den Rest

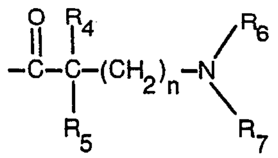

darstellt, mit einer Base wie einem Alkali-$C_1$—$C_4$-Alkoxid, z.B. Methoxid, Äthoxid oder t-Butoxid in Gegenwart von Lösungsmitteln, z.B. aromatischen Kohlenwasserstoffen wie Benzol, Toluol oder Äthern wie beispielsweise Tetrahydrofuran oder Dioxan über einen Zeitraum von 2 bis 12 Stunden erhalten werden. Die Reaktion kann durch Erwärmen des Reaktionsgemisches bis zum Siedepunkt des verwendeten Lösungsmittels beschleunigt oder beendet werden. Das Produkt wird aus dem Reaktionsgemisch durch Eindampfen im Vakuum, anschließende Extraktion des Rückstands mit einem Lösungsmittel wie Methylenchlorid oder Äthylacetat, Waschen des Extrakts mit Wasser, Trocknen über Trocknungsmitteln wie wasserfreiem Natriumsulfat und nochmaliges Eindampfen im Vakuum isoliert. Die Reinigung erfolgt mittels Chromatographie und/oder Kristallisation.

Die Verbindungen der vorliegenden Erfindung und deren Salze weisen die pharmakologischen Eigenschaften auf, welche den bereits bekannten polyoxygenierten Labdanen und deren Derivaten zugeschrieben werden, insbesondere aber abhängig von der Art und Lokalisation der Substituenten eine selektive positiv-inotrope Wirkung, blutdrucksenkende Wirkung sowie die Fähigkeit, den Augeninnendruck zu senken, auf. Dies wird aus den Ergebnissen der nachfolgenden pharmakologischen Untersuchungen ersichtlich, die zur Beurteilung der Substanzen der vorliegenden Erfindung und ihrer Salze angestellt wurden.

Positiv-inotrope Wirkung

Es wurden die beiden nachfolgenden Methoden benutzt.

1. *Isoliertes Meerschweinchenherz (Langendorff-Methode)*

Es wird ein Meerschweinchen männlichen oder weiblichen Geschlechts mit einem Gewicht zwischen 300 und 400 g getötet und das Herz mit einem kleinen Teil der Aorta entnommen. Das Herz wird umgehend in einen Perfusionsapparat gebracht, in dem die Aorta auf der Glaskanüle fixiert wird. Die aus oxygenierter Ringerlösung bestehende Perfusionslösung wird bei konstantem Druck aus dem Behälter zugeführt, der durch aus dem Thermostat zirkulierendes Wasser auf 37°C angewärmt wird. Die zu untersuchenden Verbindungen der Erfindung werden zur Herstellung einer Lösung mit bekannter Konzentration unter Verwendung stöchiometrischer 0,1 n HCl bzw. deren Salze direkt in Wasser gelöst. Die erhaltene Lösung wird durch eine Polyäthylenkanüle direkt in die Aorta injiziert und die Wirkungen auf die Kontraktionskraft, Herzfrequenz und Koronardurchblutung auf einem 3-Kanal-Physioscribe-Rekorder aufgezeichnet. Die prozentuale Zu- oder Abnahme der inotropen Wirkung wird nach dem intialen Ausgangswert berechnet.

2. *Elektrisch stimulierter linker Vorhof*

Es wird ein Meerschweinchen männlichen oder weiblichen Geschlechts mit einem Gewicht von 400 g getötet, das Herz entnommen und bei Zimmertemperatur in Ringerlösung eingebracht. Dann wird der linke Vorhof isoliert, an einem Organhalter befestigt und das Präparat in ein Organbad mit Ringerlösung von Zimmertemperatur gebracht. In das Organbad wird ein lebhafter Strom eines Gemisches von Kohlendioxid und Sauerstoff (95% $O_2$ und 5% $CO_2$) geleitet. Dann wird der linke Vorhof elektrisch stimuliert. Anschließend wird aus einer der erfindungsgemäßen Verbindungen oder ihrem Salz gegebenenfalls unter Verwendung von stöchiometrischer wäßriger 0,1 n HCl-Lösung eine wäßrige Lösung bekannter Konzentrationen hergestellt.

Diese Lösung wird dem Bad zugesetzt und die Kontraktionskraft des Vorhofes 7 bis 10 Minuten lang auf einem 4-Kanal-Kohden-Rekorder registriert. Die Wirkung wird aus den ermittelten Daten als $EC_{50}$ berechnet angegeben.

3. *In vivo-Tests am narkotisierten Hund*

Beagle-Hunde beiderlei Geschlechts mit einem Gewicht zwischen 10 und 15 kg werden mit Na-Pentobarbitol (35 mg/kg, i.v.) narkotisiert. Nach Einführen eines Endotrachealtubus werden die Femoralarterie und -vene mit einer Kanüle zur Messung des Blutdrucks bzw. zur Verabreichung von Medikamenten versehen. Der Druck des linken Ventrikels wird mit Hilfe eines Millar-Katheters gemessen und dp/dt max wird berechnet mit einem Hugo-Sachs-Differentiometer. Die Herzfrequenz wird mit Hilfe eines Kardiotachometers aus ECG-Signalen bestimmt. Alle kontinuierlich gemessen Parameter werden mit einem Hellige-Sechs-Kanal-Recorder aufgezeichnet.

Die zu untersuchenden erfindungsgemäßen Verbindungen werden in destilliertem Wasser gelöst. In allen Experimenten werden 1%ige Lösungen verwendet. Die Lösungen werden in die Femoralvene injiziert und die Dosis-Wirkung-Relation berechnet.

Die für repräsentative erfindungsgemäße Verbindungen ermittelten Resultate sind in der nachfolgenden Tabelle I dargestellt:

**Tabelle I**

| Verbindung 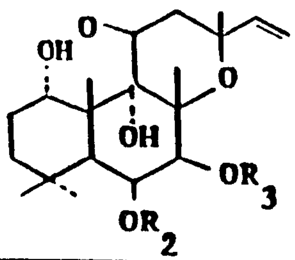 | | Isoliertes Meerschweinchenherz | | | | Meerschweinchen-Vorhof $EC_{50}$ µg/ml | Narkotisierter Hund dp/dt max $ED_{50}$ (mg/kg)i.v. |
|---|---|---|---|---|---|---|---|
| | | Dosis µg | % Zunahme der | | | | |
| | | | Inotropen Wirkung | Chronotropen Wirkung | Koronardurchblutung | | |
| $R_2$ | $R_3$ | | | | | | |
| $-CO-CH_2-N$ 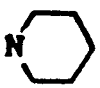 | H | 10 | 32 | 27 | 40 | 2,3 | 0,052 |
| $-CO-CH(CH_3)-N$ 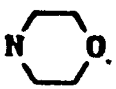 | H | 10 | 45 | 20 | 11 | 4,7 | - |

**Tabelle I (Forts.)**

| $R_2$ | $R_3$ | Isoliertes Meerschweinchenherz Dosis µg | % Zunahme der Inotropen Wirkung | Chronotropen Wirkung | Koronardurchblutung | Meerschweinchen-Vorhof $EC_{50}$ µg/ml | Narkotisierter Hund dp/dt max $ED_{50}$ (mg/kg)i.v. |
|---|---|---|---|---|---|---|---|
| 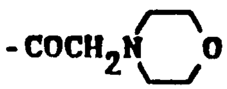 HCl | H | 30 | 23 | 34 | 35 | 0,5 | 0,11 |
| 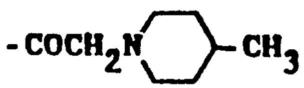 1 HCl | H | - | - | - | - | 2,6 | 0,39 |
| 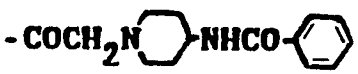 | H | - | - | - | - | 1,2 | - |
| 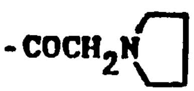 | H | - | - | - | - | 3 | - |

Tabelle I (Forts.)

| $R_2$ | $R_3$ | Isoliertes Meerschweinchenherz: Dosis µg | % Zunahme der Inotropen Wirkung | % Zunahme der Chronotropen Wirkung | % Zunahme der Koronardurchblutung | Meerschweinchen-Vorhof $EC_{50}$ µg/ml | Narkotisierter Hund dp/dt max $ED_{50}$ (mg/kg)i.v. |
|---|---|---|---|---|---|---|---|
| -$COCH_2$-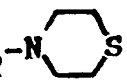 HCl.1.5$H_2O$ | H | | - | - | - | 1,5 | >3 |
| $COCH_2$-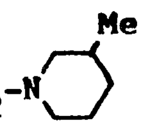 1.5$H_2O$ | H | | - | - | - | 30 | - |
| -$CO(CH_2)_4$-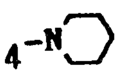 HCl | H | - | - | - | - | 30 | - |

Messung des Augeninnendruckes am wachen Kaninchen

Für die Untersuchung wird ein männliches oder weibliches Kaninchen mit einem Gewicht von 2 bis 3 kg ausgewählt. Der Augeninnendruck (IOP) wird nach vorheriger Hornhautanästhesie mit 2% Novocain-Lösung mit einem Schioetz-Tonometer gemessen. Zur Herstellung einer 2 %igen wäßrigen Lösung wird eine Verbindung der Formel I unter Verwendung von stöchiometrischer wäßriger 0,1 n HCl-Lösung oder ein Salz einer solchen Verbindung direkt in Wasser gelöst. Nach Ermittlung des Ausgangswertes werden 100 µl der 2 %igen Lösung der Testsubstanz in ein Auge, das Vehikel in das andere Auge instilliert. Der IOP wird in Abständen von 0,5, 1, 2, 3, 4 und 5 Stunden gemessen. Die prozentuale Abnahme des IOP wird anhand des Ausgangswertes berechnet.

Die Ergebnisse für die an diesem Modell getesteten repräsentativen Verbindungen dieser Erfindung sind in der nachfolgenden Tabelle aufgeführt:

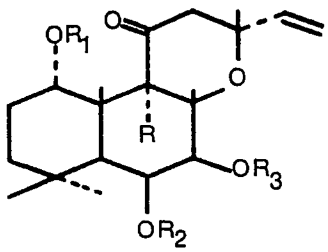

I

| $R_1$ | $R_2$ | $R_3$ | IOP-senkende Wirkung Dosis % | Senkung des IOP | Dauer (Std.) |
|---|---|---|---|---|---|
| H | 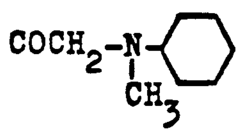 | H | 2 | 27.5 | 6 |
| H | 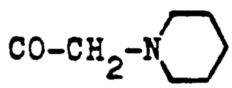 | H | 2 | 0 | - |
| H | 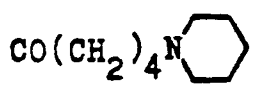 | H | 1 | 23 | 6 |

Blutdrucksenkung bei wachen hypertonen Ratten

Die blutdrucksenkende Wirkung wird bei wachen Ratten mit spontaner Hyperonie (SH) ermittelt. Eingesetzt werden dazu männliche SH-Ratten (250—300 g, 12—16 Wochen alt) des Wistar-Okamoto-Stammes aus eigener Züchtung. Vor der Blutdruckmessung werden die Ratten 10 Minuten bei 37°C in einer Wärmekammer aufgewärmt. Der systolische Blutdruck wird bei den wachen Ratten mittels Schwanzmanschetten-Methode gemessen, wobei ein piezoelektrischer Kristall zur Anzeige des Druckpulses, ein aneroides Manometer zur Druckmessung und ein Kardioskop (BPL-India) zur Anzeige des Verschwindens und/oder Auftretens des Druckpulses verwendet wird.

Die zu testenden erfindungsgemäßen Verbindungen werden unter Verwendung von stöchiometrischer 0,1 n HCl-Lösung bzw. die Salze direkt in Wasser gelöst und in dieser Form den SH-Ratten 5 Tage lang einmal täglich in einer Dosierung von 25 mg/kg oral verabreicht. Eine Gruppe dient als Kontrolle und erhält das Vehikel. Der systolische Blutdruck wird vor der ersten Verabreichung des Medikamentes und 2 Stunden danach registriert. Eine Senkung des systolischen Blutdruckes ist die Differenz zwischen dem Blutdruck nach der Behandlung und dem Ausgangswert.

Die Resultate für die repräsentativen Verbindungen der Erfindung und deren Salze sind in der nachfolgenden Tabelle aufgeführt:

| Verbindung 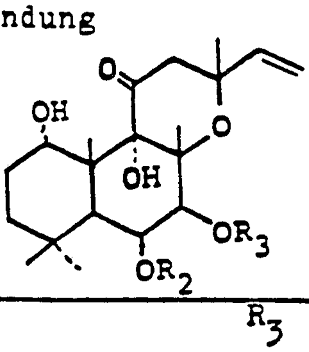 | | Dosis (mg/kg) oral | Senkung des systolischen Blutdruckes |
|---|---|---|---|
| $R_2$ | $R_3$ | | |
| $-COCH_2-$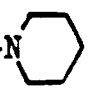 | H | 25 | 14 |

Wirksame Dosen der erfindungsgemäßen Verbindungen können z.B. oral zusammen mit pharmakologisch unbedenklichen Hilfs- und Trägerstoffen in Form von Gelatinekapseln, Tabletten, Elixieren, Suspensionen, Sirups usw. verabreicht werden. Diese Zubereitungen enthalten mindestens 0,5%, im allgemeinen jedoch zwischen etwa 4 und 70% der wirksamen Verbindung. Die Menge der wirksamen Verbindung in solchen Zubereitungen wird so bemessen, daß man eine geeignete Dosis erhält. Bevorzugt enthält eine solche Dosiseinheit zur oralen Verabreichung zwischen 0,1—30 mg einer wirksamen Verbindung.

Zum Zwecke der parenteralen oder topischen Verabreichung werden Zubereitung wie Lösungen, Suspensionen, Tinkturen, Salben oder Cremes hergestellt, welche mindestens 0,01%, im allgemeinen jedoch zwischen etwa 0,5 und 5 (Gew.-)% der wirksamen Verbindung enthalten. Bevorzugt enthält eine Dosiseinheit für diesen Zweck zwischen 0,01 und 10 mg einer wirksamen Verbindung.

Die Lösungen oder Suspensionen für die parenterale Verabreichung können die folgenden Komponenten enthalten: ein steriles Verdünnungsmittel, z.B. Wasser, Kochsalzlösung, pflanzliche Öle, Polyethylenglykole, Glycerin, Propylenglykol oder andere synthetische Lösungsmittel; antibakterielle Mittel wie Benzylalkohol; Antioxidantien wie Ascorbinsäure oder Natriumbisulfit; Cheliermittel wie EDTA und Puffer wie Acetate, Citrate oder Phosphate.

Die Erfindung wird durch die nachfolgenden Beispiele illustriert.

Referenzbeispiel 1

8,13-Epoxy-1α-piperidinoacetoxy-6β-7β-9α-trihydroxy-labd-14-en-11-on

Eine Lösung aus Chloracetylchlorid (0,24 ml) in Dichlormethan (2 ml) wurde in einer Mischung von 8,13-Epoxy-1α,6β-7β-9α-tetrahydroxy-labd-14-en-11-on (1,0 g), gelöst in Dichlormethan (10 ml) und trockenem Pyridin (0,42 ml) zugesetzt, die in einem Eis-Salzbad gekühult wurde. Die Reatkionsmischung wurde 1 1/2 Stunden bei 0°C gerüht und im Vakuum eingeengt. Der Rückstand wurde mit Dichlormethan extrahiert. Die organische Schicht wurde abgetrennt, nacheinander mit verdünnter Salzsäure (10%), gesättigter Natriumbicarbonat-Lösung und Wasser gewaschen und abschließend über wasserfreiem Natriumsulfat getrocknet, Der Extrakt wurde bis auf einen Rückstand (1,1 g) eingeengt. Der Rückstand wurde mit Piperidin (10 ml) behandelt und die Mischung zwei Stunden am Rückfluß gekocht. Das Reaktionsgemisch wurde dann im Vakuum eingeeingt und der Rückstand mit Äthylacetat extrahiert. Der Extrakt wurde mit Wasser gewaschen und über wasserfreiem Natriumsulfat getrocknet. Der Rückstand nach Eindampfen des Extraktes bestand aus einer Mischung von Produkten, wie durch Dünnschichtchromatographie nachgewiesen. Die Reinigung des Produktgemisches durch Chromatographie über einer Kieselgel-Säule und die Elution mit Äthylacetat: Petrolether (1:1) als Eluens lieferte das gewünschte 8,13-Epoxy-1α-piperidinoacetoxy-6β,7β-9α-trihydroxy-labd-14-en-11-on mit einem Schmelzpunkt von 89—91°C. Die übrigen Produkte der Mischung wurden ebenfalls isoliert. Die Einzelheiten werden in Referenzbeispielen 3 und Beispiel 1 beschrieben.

Referenzbeispiel 2

8,13-Epoxy-1α-piperidinoacetoxy-6β,7β,9α-trihydroxy-labd-14-en-11-on-hydrochloridhemihydrat

Diäthyläther (10 ml, bei 0°C mit trockenem HCl-Gas gesättigt) wurde einer methanolischen Lösung von 8,13-Epoxy-1α-piperidinoacetoxy-6β,7β,9α-trihydroxy-labd-14-en-11-on (0,5 g in 5 ml Methanol) zugesetzt. Die Mischung wurde dann mit einem Überschuß von trockenem Diäthyläther verdünnt und anschließend die ausgefallene feste Substanz mittels Filtration abgetrennt. Die Rekristallisation der festen Substanz aus Methanol-Äther ergab 8,13-Epoxy-1α-piperidino-acetoxy-6β,7β,9α-trihydroxy-labd-en-11-on-hydrochlorid-hemihydrat mit einem Schmelzpunkt von 144—146°C.

Referenzbeispiel 3

8,13-Epoxy-7β-piperidinoacetoxy-1α,6β,9α-trihydroxy-labd-14-en-11-on

Das in Referenzbeispiel 1 beschriebene Verfahren wurde wiederholt. Das durch Reinigung mittels Chromatographie über eine Silikalgel-Säure unter Verwendung von Äthylacetat/Petroläther (1:1) als Eluenten gewonnene Produktgemisch ergab 8,13-Epoxy-1α-piperidinoacetoxy-6β,7β,9α-trihydroxy-labd-14-en-11-on, nach desen Abtrennung die weitere Elution das gewunschte 8,13-Epoxy-7β-piperidinoacetoxy-1α,6β,9α-trihydroxy-labd-14-en-11-on mit einem Schmelzpunkt von 162—164°C lieferte.

Beispiel 1

8,13-Epoxy-6β-piperidinoacetoxy-1α,7β,9α-trihydroxy-labd-14-en-11-on

*Methode 1*

Das in Referenzbeispiel 1 beschriebene Verfahren wurde wiederholt. Das erhaltene Produktgemisch wurde mittels "flash"-Säulenchromatographie über Silikagel unter Verwendung von Äthylacetat/Petroläther (1:1) als Eluenten gereinigt. Die erste eluierte Verbindung war 8,13-Epoxy-1α-piperidinoacetoxy-6β,7β,9α-trihydroxy-labd-14-en-11-on, gefolgt von der zweiten Verbindung, 8,13-Epoxy-7β-piperidinoacetoxy-1α,6β,9α-trihydroxy-labd-14-en-11-on, während die weitere Elution das Produkt 8,13-Epoxy-6β-piperidinoacetoxy-1α,7β,9α-trihydroxy-labd-14-en-11-on mit einem Schmelzpunkt von 111—113°C lieferte.

*Methode 2*

8,13-Epoxy-7β-piperidinoacetoxy-1α,6β,9α-trihydroxy-labd-14-en-11-on (0,1 M) wurde mit Natriummethoxid (0,1 M) 12 Stunden lang in trockenem Dioxan (25 ml) gerührt. Das Reaktionsgemisch wurde dann im Vakuum bei 0°C eingeengt und der Rückstand mit Dichlormethan extrahiert. Der Extrakt wurde mit Wasser gewaschen und über wasserfreiem Natriumsulfat getrocknet. Durch Einengen erhielt man einen Rückstand, der nach Reinigung mittels Flüssigchromatographie bei mittlerem Druck 8,13-Epoxy-6β-piperidinoacetoxy-1α,7β,9α-trihydroxy-labd-14-en-11-on mit einem Schmelzpunkt von 111—113°C ergab.

*Methode 3*

8,13-Epoxy-7β-piperidinoacetoxy-1α,6β,9α-trihydroxy-labd-14-en-11-on (1,0 g) wurde in trockenem Piperidin (10 ml) 12 Stunden am Rückfluß gekocht. Das Reaktionsgemisch wurde dann im Vakuum eingedampft und der Rückstand mit Essigester extrahiert. Der Extrakt wurde mit Wasser und Kochsalzlösung gewaschen und über Natriumsulfat getrocknet. Durch Einengen erhielt man einen Rückstand, der nach Reinigen durch flash-Säulenchromatographie 8,13-Epoxy-6β-piperidinoacetoxy-1α,7β,9α-trihydroxy-labd-14-en-11-on (Schmelzpunkt 111—113°C) ergab.

Die nachfolgenden Verbindungen wurden unter Verwendung geeigneter Amine anstelle von Piperidin auf die gleiche Weise hergestellt (die Hydrochloridsalze wurden nach dem in Referenzbeispiel 2 beschriebenen Verfahren hergestellt):

8,13-Epoxy-6β-morpholinoacetoxy-1α,7β,9α-trihydroxy-labd-14-en-11-on, Schmelzpunkt 210—212°C.

8,13-Epoxy-6β-morpholinoacetoxy-1α,7β,9α-trihydroxy-labd-14-en-11-on, Schmelzpunkt 152—155°C.

8,13-Epoxy-6β-pyrrolidinoacetoxy-1α,7β,9α-trihydroxy-labd-14-en-11-on, Schmelzpunkt 116—118°C.

8,13-Epoxy-6β-(N-methylpiperazinoacetoxy)-1α,7β,9α-trihydroxy-labd-14-en-11-on-dihydrochlorid, Schmelzpunkt 182—184°C.

8,13-Epoxy-6β-(N-methyl-cyclohexylaminoacetoxy)-1α,7β,9α-trihydroxy-labd-14-en-11-on, Schmelzpunkt 137—139°C.

8,13-Epoxy-6β-[α'(N-morpholino)propionoxy]-1α,7β,9α-trihydroxy-labd-14-en-11-on-dihydrochlorid, Schmelzpunkt 179—180°C.

8,13-Epoxy-6β-[α'(N-piperidino)propionoxy]-1α,7β,9α-trihydroxy-labd-14-en-11-on-dihydrochlorid, Schmelzpunkt 260—261°C.

8,13-Epoxy-6β-[α'(4N-methylpiperazino)propionoxy]-1α,7β,9α-trihydroxy-labd-14-en-11-on-dihydrochlorid, Schmelzpunkt 208—209°C.

8,13-Epoxy-6β-(4'-hydroxy-piperidinoacetoxy)-1α,7β,9α-trihydroxy-labd-14-en-11-on-hydrochlorid-dihydrat, Schmelzpunkt 187—190°C:

8,13-Epoxy-6β-(thiomorpholinoacetoxy)-1α,7β,9α-trihydroxy-labd-14-en-11-on-hydrochloridsesqui-hydrat, Schmelzpunkt 173—176°C.

8,13-Epoxy-6β(4'-methylpiperidinoacetoxy)-1α,7β,9α-trihydroxy-labd-14-en-11-on-hydrochlorid-dihydrat, Schmelzpunkt 125—128°C.

8,13-Epoxy-6β(4'-benzoylaminopiperidinoacetoxy)-1α,7β,9α-trihydroxy-labd-14-en-11-on-hemihydrat, 122—124°C.

8,13-Epoxy-6β(γ'-piperidinobutyryloxy)-1α,7β,9α-trihydroxy-labd-14-en-11-on-hydrochlorid-hemihydrat, Schmelzpunkt 178—181°C.

8,13-Epoxy-6β(diethylaminoacetoxy)-1α,7β,9α-trihydroxy-labd-14-en-11-on-monohydrochlorid-dihydrat, Schmelzpunkt 160—163°C.

8,13-Epoxy-6β(γ'-morpholinobutyryloxy)-1α,7β,9α-trihydroxy-labd-14-en-11-on-hydrochlorid-dihydrat, Schmelzpunkt 232—233°C.

8,13-Epoxy-6β(α'-anilinopropionyloxy)-1α,7β,9α-trihydroxy-labd-14-en-11-on, Schmelzpunkt 218—219°C.

8,13-Epoxy-6β(4'-hydroxy-4'-phenylpiperidinoacetoxy)-1α,7β,9α-trihydroxy-labd-14-en-11-on-hydrat, Schmelzpunkt 123—125°C.

8,13-Epoxy-6β(theophyllinoacetoxy)-1α,7β,9α-trihydroxy-labd-14-en-11-on, Schmelzpunkt 149—153°C (Zers.).

8,13-Epoxy-6β(benzylaminoacetoxy)-1α,7β,9α-trihydroxy-labd-14-en-11-on-hydrochloridmonohydrat, Schmelzpunkt 169—172°C.

8,13-Epoxy-6β(3'-methylpiperidinoacetoxy)-1α,7β,9α-trihydroxy-labd-14-en-11-on-hydrochloridsesquihydrat, Schmelzpunkt 188—190°C.

8,13-Epoxy-6β(δ'-piperidinovaleryloxy)-1α,7β,9α-trihydroxy-labd-14-en-11-on-monohydrochlorid-monohydrat. Fp. 148—150°C.

**Patentansprüche für die Vertragsstaaten: BE CH DE FR GB IT LI LU NL SE**

1. Verbindung der Formel I

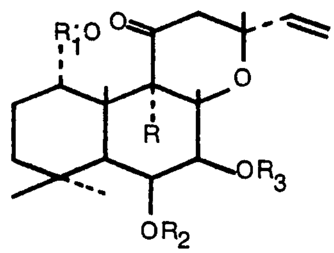

worin bedeuten:

R Wasserstoff oder Hydroxyl;

$R_2$ den Rest

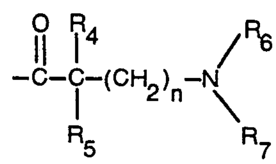

und $R_1$ und $R_3$ Wasserstoff, wobei

$R_4$ und $R_5$ gleich oder verschieden sind und Wasserstoff oder $C_1$—$C_4$-Alkyl darstellen,

n für 0 oder eine ganze Zahl von 1 bis 4 steht,

$R_6$ Wasserstoff bedeutet und

$R_7$ Wasserstoff, unsubstituiertes oder substituiertes $C_1$—$C_4$-Alkyl, Cyclohexyl, Phenyl oder Phenyl-$C_1$—$C_2$-alkyl, wobei der Phenylrest ein- oder mehrfach mit Halogen, $C_1$—$C_4$-Alkyl. $C_1$—$C_4$-Alkoxy, Nitro und/oder Trifluormethyl substituiert sein kann, Pyridin, Chinolin, gegebenenfalls substituiertes Amino, Hydroxy, $C_1$—$C_6$-Alkanoyl, $C_2$—$C_6$-Alkenoyl, Benzoyl oder Phenyl-$C_1$—$C_6$-alkanoyl, worin der Phenylrest ein- oder mehrfach mit Halogen, $C_1$—$C_4$-Alkyl, $C_1$—$C_4$-Alkoxy, Nitro oder Trifluormethyl substituiert sein kann, Di-$C_1$—$C_4$-alkylamino, Carbamyl, $C_1$—$C_4$-Alkoxycarbonyl, $C_1$—$C_4$-Alkoxycarbonyl-$C_1$—$C_4$-alkyl, oder

$R_6$ und $R_7$ die gleiche Bedeutung haben und gegebenenfalls substituiertes $C_1$—$C_4$-Alkyl oder Phenyl oder Phenyl-$C_1$—$C_2$-Alkyl darstellen, wobei die Phenylreste wie oben angegeben substituiert sein können, oder

$R_6$ $C_1$—$C_4$-Alkyl bedeutet und $R_7$ substituiertes $C_1$—$C_4$-Alkyl oder Cyclohexyl, Phenyl-$C_1$—$C_4$-alkyl, worin der Phenylrest ein- oder mehrfach mit Halogen, $C_1$—$C_4$-Alkyl, $C_1$—$C_4$-Alkoxy, Nitro und/oder Trifluormethyl substituiert sein kann, oder Di-$C_1$—$C_4$-alkylamino-$C_1$—$C_4$-alkyl darstellt, oder

$R_6$ und $R_7$ zusammen mit dem N-Atom, an das sie gebunden sind, den Piperidin-, Pyrrolidin-, Morpholin-, Piperazin-, Thiomorpholin-, Imidazol- oder Theophyllin-Rest darstellen, wobei die genannten Reste ein- oder mehrfach mit $C_1$—$C_4$-Alkyl, $C_1$—$C_4$-Alkoxy, Phenyl, Phenyl-$C_1$—$C_2$-alkyl, Hydroxy, Amino- oder Hydroxy-$C_1$—$C_4$-alkyl oder substituierte Phenyl- oder Aminogruppen substituiert sein können, sowie deren pharmakologisch unbedenklichen Salze.

2. Verbindungen gemäß Anspruch 1, worin R Hydroxy ist und $R_2$ den Rest

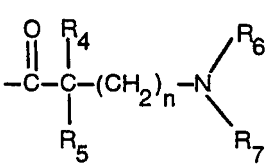

bedeutet, worin $R_4$ und $R_5$ Wasserstoff oder $R_4$ Wasserstoff und $R_5$ $C_1$—$C_4$-Alkyl, $R_6$ $C_1$—$C_4$-Alkyl und $R_7$ Cyclohexyl, oder $R_6$ und $R_7$ zusammen mit dem N-Atom, an das sie gebunden sind, den Piperidino-,

Morpholino-, Piperazino- oder Pyrrolidino-Rest darstellen, und n 0 ist, sowie ihre pharmakologisch unbedenklichen Salze.

3. Arzneimittel, gekennzeichnet durch einen Gehalt an einer Verbindung der Formel I gemäß Anspruch 1 und/oder einem pharmakologisch unbedenklichen Salz.

4. Verwendung einer Verbindung der Formel I und/oder eines pharmakologisch unbedenklichen Salzes zur Herstellung von Arzneimitteln.

5. Verfahren zur Herstellung einer Verbindung der Formel I, worin R, $R_1$ bis $R_7$ und n die in Anspruch 1 angegebene Bedeutung besitzen sowie deren pharmazeutisch unbedenklichen Salze, dadurch gekennzeichnet, daß man

a) eine Verbindung der Formel II

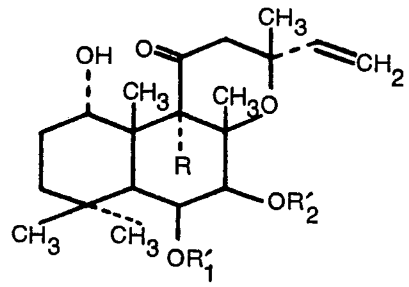

worin R Wasserstoff oder Hydroxy bedeutet und $R'_1$ und $R'_2$ für Wasserstoff stehen, mit einer Verbindung der Formel III

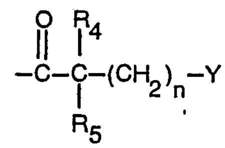

worin X und Y Halogen bedeuten und $R_4$, $R_5$ und n die in Anspruch 1 genannten Bedeutungen haben, in Gegenwart einer Base umsetzt, und

b) die erhaltene Mischung von Verbindungen der Formel IV

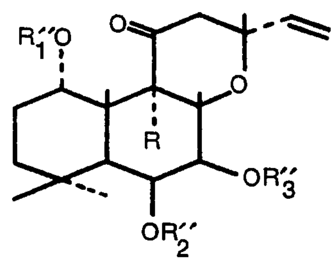

worin $R''_1$, $R''_2$ und $R''_3$ Wasserstoff oder den Rest

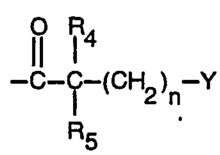

bedeuten, wobei mindestens einer der Substituenten $R''_1$ bis $R''_3$ den genannten Rest darstellt, mit einer Verbindung der Formel V

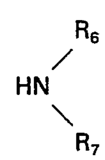

worin $R_6$ und $R_7$ die in Anspruch 1 genannten Bedeutungen haben, umsetzt, und aus dem erhaltenen Gemisch die Verbindungen der Formel I auf übliche Weise isoliert, und

c) gegebenenfalls eine Verbindung der Formel I, worin R Wasserstoff oder Hydroxy bedeutet, $R_1$ Wasserstoff, $R_2$ Wasserstoff und $R_3$ den Rest

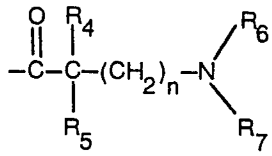

worin $R_4$ bis $R_7$ und n die in Anspruch 1 genannten Bedeutungen haben, darstellt, mit einem Alkali-$C_1$—$C_4$-Alkoxid umsetzt zu einer Verbindung der Formel I, worin $R_1$ Wasserstoff, $R_2$ den Rest

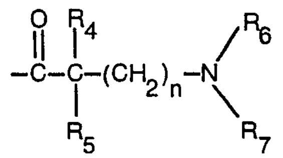

und $R_3$ Wasserstoff bedeutet.

6. Verfahren nach Anspruch 5, dadurch gekennzeichnet, daß man die Umsetzung einer Verbindung der Formel I mit einer Verbindung der Formel III in Gegenwart einer organischen Stickstoff-Base bei Temperatur von 0°C bis Raumtemperatur und in einem aromatische Kohlenwasserstoff, einem Äther oder in einem halogenierten Kohlenwasserstoff, die Umsetzung einer Verbindung der Formel IV mit einer Verbindung der Formel V 1 bis 6 Stunden bei Temperaturen von Raumtemperatur bis zu 150°C mit oder ohne Lösungsmitteln durchführt.

7. Verfahren nach Anspruch 5, dadurch gekennzeichnet, daß man eine Verbindung der Formel I mit Natriummethoxyd, Natriummäthoxid oder Natrium-t-butoxid in Gegenwart von aromatischen Kohlenwasserstoffen oder Äthern als Lösungsmittel über einen Zeitraum von 2 bis 12 Stunden behandelt.

**Patentansprüche für die Vertragsstaaten: AT ES**

1. Verfahren zur Herstellung von Verbindungen der Formel I

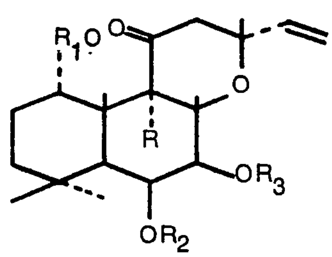

worin bedeuten:

R Wasserstoff oder Hydroxyl;

$R_2$ den Rest

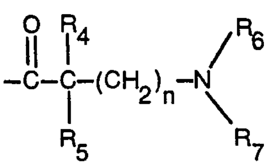

und

$R_1$ und $R_3$ Wasserstoff, wobei

$R_4$ und $R_5$ gleich oder verschieden sind und Wasserstoff oder $C_1$—$C_4$-Alkyl darstellen,

n für 0 oder eine ganze Zahl von 1 bis 4 steht,

$R_6$ Wasserstoff bedeutet und

$R_7$ Wasserstoff, unsubstituiertes oder substituiertes $C_1$—$C_4$-Alkyl, Cyclohexyl, Phenyl oder Phenyl-$C_1$—$C_2$-alkyl, wobei der Phenylrest ein- oder mehrfach mit Halogen, $C_1$—$C_4$-Alkyl, $C_1$—$C_4$-Alkoxy, Nitro und/oder Trifluormethyl substituiert sein kann, Pyridin, Chinolin, gegebenenfalls substituiertes Amino, Hydroxy, $C_1$—$C_6$-Alkanoyl, $C_2$—$C_6$-Alkenoyl, Benzoyl oder Phenyl-$C_1$—$C_6$-alkanoyl, worin der Phenylrest ein- oder mehrfach mit Halogen, $C_1$—$C_4$-Alkyl, $C_1$—$C_4$-Alkoxy, Nitro oder Trifluormethyl substituiert sein kann, Di-$C_1$—$C_4$-alkylamino, Carbamyl, $C_1$—$C_4$-Alkoxycarbonyl, $C_1$—$C_4$-Alkoxycarbonyl-$C_1$—$C_4$-alkyl, oder

$R_6$ und $R_7$ die gleiche Bedeutung haben und gegebenenfalls substituiertes $C_1$—$C_4$-Alkyl oder Phenyl oder Phenyl-$C_1$—$C_2$-Alkyl darstellen, wobei die Phenylreste wie oben angegeben substituiert sein können, oder

$R_6$ $C_1$—$C_4$-Alkyl bedeutet und $R_7$ substituiertes $C_1$—$C_4$-Alkyl oder Cyclohexyl, Phenyl-$C_1$—$C_4$-alkyl, worin der Phenylrest ein- oder mehrfach mit Halogen, $C_1$—$C_4$-Alkyl, $C_1$—$C_4$-Alkoxy, Nitro und/oder Trifluoromethyl substituiert sein kann, oder Di-$C_1$—$C_4$-alkylamino-$C_1$—$C_4$-alkyl darstellt, oder

$R_6$ und $R_7$ zusammen mit dem N-Atom, an das sie gebunden sind, den Piperidin-, Pyrrolidin-, Morpholin-, Piperazin-, Thiomorpholin-, Imidazol- oder Theophyllin-Rest darstellen, wobei die genannten Reste ein- oder mehrfach mit $C_1$—$C_4$-Alkyl, $C_1$—$C_4$-Alkoxy, Phenyl, Phenyl-$C_1$—$C_2$-alkyl, Hydroxy, Amino- oder Hydroxy-$C_1$—$C_4$-alkyl oder substituierte Phenyl- oder Aminogruppen substituiert sein können, sowie deren pharmakologisch unbedenklichen Salze, dadurch gekennzeichnet, daß man

a) eine Verbindung der Formel II

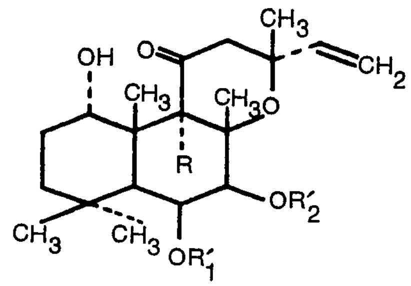

worin R Wasserstoff oder Hydroxy bedeutet und $R'_1$ und $R'_2$ für Wasserstoff stehen, mit einer Verbindung der Formel III

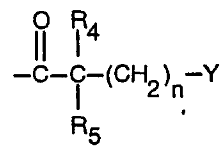

worin X und Y Halogen bedeuten und $R_4$, $R_5$ und n die in den genannten Bedeutungen haben, in Gegenwart einer Base umsetzt, und

b) die erhaltene Mischung von Verbindungen der Formel IV

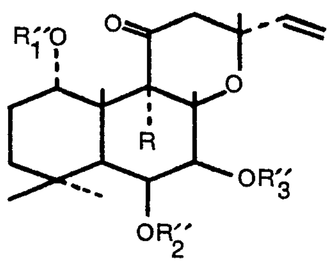

worin $R''_1$, $R''_2$ und $R''_3$ Wasserstoff oder den Rest

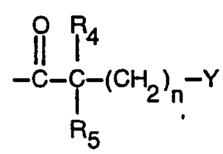

bedeuten, wobei mindestens einer der Substituenten $R''_1$ bis $R''_3$ den genannten Rest darstellt, mit einer Verbindung der Formel V

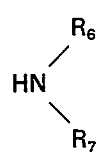

worin $R_6$ und $R_7$ die ober genannten Bedeutungen haben, umsetzt, und aus dem erhaltenen Gemisch die Verbindungen der Formel I auf übliche Weise isoliert, und

c) gegebenenfalls eine Verbindung der Formel I, worin R Wasserstoff oder Hydroxy bedeutet, $R_1$ Wasserstoff, $R_2$ Wasserstoff und $R_3$ den Rest

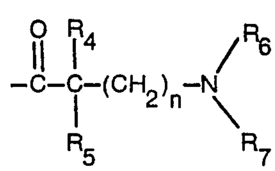

worin $R_4$ bis $R_7$ und n die ober genannten Bedeutungen haben, darstellt, mit einem Alkali-$C_1$—$C_4$-Alkoxid umsetzt zu einer Verbindung der Formel I, worin $R_1$ Wasserstoff, $R_2$ den Rest

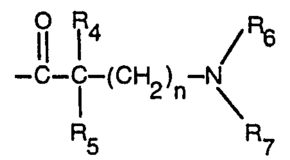

und $R_3$ Wasserstoff bedeutet.

2. Verfahren nach Anspruch 1, dadurch gekennzeichnet, daß man die Umsetzung einer Verbindung der Formel I mit einer Verbindung der Formel III in Gegenwart einer organischen Stickstoff-Base bei Temperatur von 0°C bis Raumtemperatur und in einem aromatischen Kohlenwasserstoff, einem Äther oder in einem halogenierten Kohlenwasserstoff, die Umsetzung einer Verbindung der Formel IV mit einer Verbindung der Formel V 1 bis 6 Stunden bei Temperaturen von Raumtemperatur bis zu 150°C mit oder ohne Lösungsmitteln durchführt.

3. Verfahren nach Anspruch 1, dadurch gekennzeichnet, daß man eine Verbindung der Formel I mit Natriummethoxyd, Natriummäthoxid oder Natrium-t-butoxid in Gegenwart von aromatischen Kohlenwasserstoffen oder Äthern als Lösungsmittel über einen Zeitraum von 2 bis 12 Stunden behandelt.

4. Verfahren gemäß Ansprüchen 1 bis 3, dadurch gekennzeichnet, daß R Hydroxy ist und $R_2$ den Rest

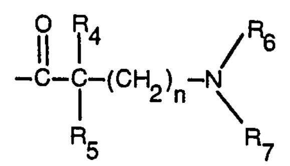

bedeutet, worin $R_4$ und $R_5$ Wasserstoff oder $R_4$ Wasserstoff und $R_5$ $C_1$—$C_4$-Alkyl, $R_6$ $C_1$—$C_4$-Alkyl und $R_7$ Cyclohexyl, oder $R_6$ und $R_7$ zusammen mit dem N-Atom, an das sie gebunden sind, den Piperidino-, Morpholino-, Piperazino- oder Pyrrolidino-Rest darstellen, und n 0 ist.

5. Verwendung einer Verbindung der Formel I und/oder eines pharmakologisch unbedenklichen Salzes zur Herstellung von Arzneimitteln.

**Patentansprüche für den Vertragsstaat: GR**

1. Verbindung der Formel I

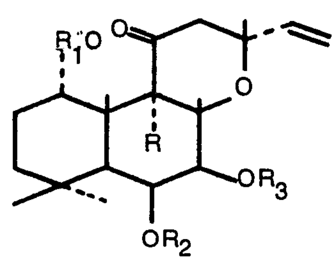

worin bedeuten:

R Wasserstoff oder Hydroxyl;

$R_2$ den Rest

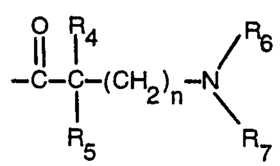

und $R_1$ und $R_3$ Wasserstoff, wobei

$R_4$ und $R_5$ gleich oder verschieden sind und Wasserstoff oder $C_1$—$C_4$-Alkyl darstellen,

n für 0 oder eine ganze Zahl von 1 bis 4 steht,

$R_6$ Wasserstoff bedeutet und

$R_7$ Wasserstoff, unsubstituiertes oder substituiertes $C_1$—$C_4$-Alkyl, Cyclohexyl, Phenyl oder Phenyl-$C_1$—$C_2$-alkyl, wobei der Phenylrest ein- oder mehrfach mit Halogen, $C_1$—$C_4$-Alkyl, $C_1$—$C_4$-Alkoxy, Nitro und/oder Trifluormethyl substituiert sein kann, Pyridin, Chinolin, gegebenenfalls substituiertes Amino, Hydroxy, $C_1$—$C_6$-Alkanoyl, $C_2$—$C_6$-Alkenoyl, Benzoyl oder Phenyl-$C_1$—$C_6$-alkanoyl, worin der Phenylrest ein- oder mehrfach mit Halogen, $C_1$—$C_4$-Alkyl, $C_1$—$C_4$-Alkoxy, Nitro oder Trifluormethyl substituiert sein kann, Di-$C_1$—$C_4$-alkylamino, Carbamyl, $C_1$—$C_4$-Alkoxycarbonyl, $C_1$—$C_4$-Alkoxycarbonyl-$C_1$—$C_4$-alkyl, oder

$R_6$ und $R_7$ die gleiche Bedeutung haben und gegebenenfalls substituiertes $C_1$—$C_4$-Alkyl oder Phenyl oder Phenyl-$C_1$—$C_2$-Alkyl darstellen, wobei die Phenylreste wie oben angegeben substituiert sein können, oder

$R_6$ $C_1$—$C_4$-Alkyl bedeutet und $R_7$ substituiertes $C_1$—$C_4$-Alkyl oder Cyclohexyl, Phenyl-$C_1$—$C_4$-alkyl, worin der Phenylrest ein- oder mehrfach mit Halogen, $C_1$—$C_4$-Alkyl, $C_1$—$C_4$-Alkoxy, Nitro und/oder Trifluormethyl substituiert sein kann, oder Di-$C_1$—$C_4$-alkylamino-$C_1$—$C_4$-alkyl darstellt, oder

$R_6$ und $R_7$ zusammen mit dem N-Atom, an das sie gebunden sind, den Piperidin-, Pyrrolidin-, Morpholin-, Piperazin-, Thiomorpholin-, Imidazol- oder Theophyllin-Rest darstellen, wobei die genannten Reste ein- oder mehrfach mit $C_1$—$C_4$-Alkyl, $C_1$—$C_4$-Alkoxy, Phenyl, Phenyl-$C_1$—$C_2$-alkyl, Hydroxy, Amino- oder Hydroxy-$C_1$—$C_4$-alkyl oder substituierte Phenyl- oder Aminogruppen substituiert sein können, sowie deren pharmakologisch unbedenklichen Salze.

2. Verbindungen gemäß Anspruch 1, worin R Hydroxy ist und $R_2$ den Rest

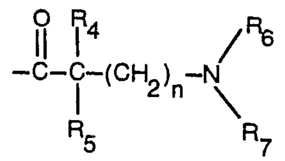

bedeutet, worin $R_4$ und $R_5$ Wasserstoff oder $R_4$ Wasserstoff und $R_5$ $C_1$—$C_4$-Alkyl, $R_6$ $C_1$—$C_4$-Alkyl und $R_7$ Cyclohexyl, oder $R_6$ und $R_7$ zusammen mit dem N-Atom, an das sie gebunden sind, den Piperidino-, Morpholino-, Piperazino- oder Pyrrolidino-Rest darstellen, und n 0 ist, sowie ihre pharmakologisch unbedenklichen Säureadditionssalze.

3. Verwendung einer Verbindung der Formel I und/oder eines pharmakologisch unbedenklichen Salzes zur Herstellung von Arzneimitteln.

4. Verfahren zur Herstellung einer Verbindung der Formel I, worin R, $R_1$ bis $R_7$ und n die in Anspruch 1 angegebene Bedeutung besitzen sowie deren pharmazeutisch unbedenklichen Salze, dadurch gekennzeichnet, daß man

a) eine Verbindung der Formel II

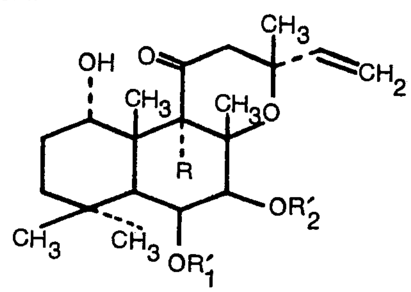

worin R Wasserstoff oder Hydroxy bedeutet und $R'_1$ und $R'_2$ für Wasserstoff stehen, mit einer Verbindung der Formel III

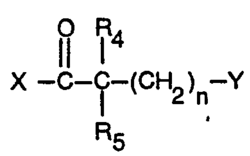

worin X und Y Halogen bedeuten und $R_4$, $R_5$ und n die in Anspruch 1 genannten Bedeutungen haben, in Gegenwart einer Base umsetzt, und

b) die erhaltene Mischung von Verbindungen der Formel IV

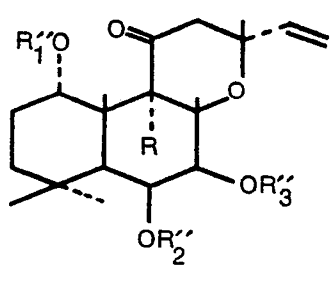

worin $R''_1$, $R''_2$ und $R''_3$ Wasserstoff oder den Rest

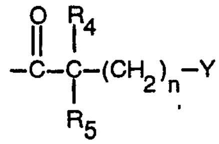

bedeuten, wobei mindestens einer der Substituenten $R''_1$ bis $R''_3$ den genannten Rest darstellt, mit einer Verbindung der Formel V

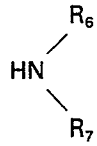

worin $R_6$ und $R_7$ die in Anspruch 1 genannten Bedeutungen haben, umsetzt, und aus dem erhaltenen Gemisch die Verbindungen der Formel I auf übliche Weise isoliert, und

c) gegebenenfalls eine Verbindung der Formel I, worin R Wasserstoff oder Hydroxy bedeutet, $R_1$ Wasserstoff, $R_2$ Wasserstoff und $R_3$ den Rest

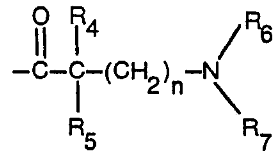

worin $R_4$ bis $R_7$ und n die in Anspruch 1 genannten Bedeutungen haben, darstellt, mit einem Alkali-$C_1$—$C_4$-Alkoxid umsetzt zu einer Verbindung der Formel I, worin $R_1$ Wasserstoff, $R_2$ den Rest

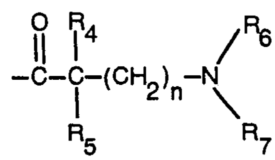

und $R_3$ Wasserstoff bedeutet.

5. Verfahren nach Anspruch 4, dadurch gekennzeichnet, daß man die Umsetzung einer Verbindung der Formel I mit einer Verbindung der Formel III in Gegenwart einer organischen Stickstoff-Base bei Temperatur von 0°C bis Raumtemperatur und in einem aromatischen Kohlenwasserstoff, einem Äther oder in einem halogenierten Kohlenwasserstoff, die Umsetzung einer Verbindung der Formel IV mit einer Verbindung der Formel V 1 bis 6 Stunden bei Temperaturen von Raumtemperatur bis zu 150°C mit oder ohne Lösungsmitteln durchführt.

6. Verfahren nach Anspruch 4, dadurch gekennzeichnet, daß man eine Verbindung der Formel I mit Natriummethoxyd, Natriummäthoxid oder Natrium-t-butoxid in Gegenwart von aromatischen Kohlenwasserstoffen oder Äthern als Lösungsmittel über einen Zeitraum von 2 bis 12 Stunden behandelt.

**Revendications pour les Etats contractants: BE CH DE FR GB IT LI LU NL SE**

1. Composés de formule I

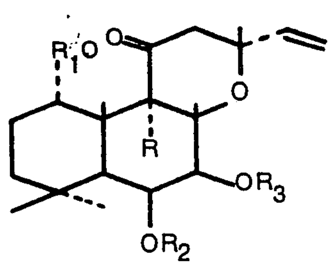

dans laquelle

R représente un atome d'hydrogène ou le groupe hydroxy;

$R_2$ représente le radical

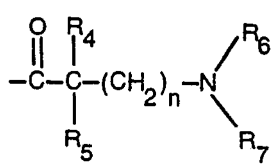

et

$R_1$ et $R_3$ représentent des atomes d'hydrogène;

$R_4$ et $R_5$ étant identiques ou différents, et représentant un atome d'hydrogène ou un groupe alkyle en $C_1$—$C_4$,

n étant zéro ou un nombre entier allant de 1 à 4,

$R_6$ représentant un atome d'hydrogène, et

$R_7$ représentant un atome d'hydrogène, un radical alkyle en $C_1$—$C_4$ substitué ou non substitué, cyclohexyle, phényle ou phényl-alkyle($C_1$—$C_2$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, un radical pyridine, quinoléine, amino éventuellement substitué, hydroxy, alcanoyle en $C_1$—$C_6$, alcénoyle en $C_2$—$C_6$, benzoyle ou phényl-alcanoyle-($C_1$—$C_6$), le reste phényle pouvant être une ou plusieurs fois substitué par un ou des atomes d'halogène ou groupes alkyle en $C_1$—$C_4$, alcoxy en $C_1$—$C_4$, nitro ou trifluorométhyle, un radical dialkyl($C_1$—$C_4$)-amino, carbamoyle, alcoxy($C_1$—$C_4$)-carbonyle, alcoxy($C_1$—$C_4$)-carbonyle, alcoxy($C_1$—$C_4$)-carbonyl-alkyle($C_1$—$C_4$), ou

$R_6$ et $R_7$ ont la même signification et représentent un radical alkyle en $C_1$—$C_4$ éventuellement substitué ou phényle ou phényl-alkyle($C_1$—$C_2$), les restes phényle pouvant être substitués comme indiqué plus haut, ou

$R_6$ représente un radical alkyle en $C_1$—$C_4$ et $R_7$ représente un radical alkyle en $C_1$—$C_4$ substitué ou cyclohexyle, phényl-alkyle($C_1$—$C_4$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, ou un radical dialkyl($C_1$—$C_4$)-aminoalkyle($C_1$—$C_4$), ou

$R_6$ et $R_7$ représentent ensemble, conjointement avec l'atome d'azote auquel il sont liés, le radical pipéridino, pyrrolidino, morpholino, pipérazino, thiomorpholino, imidazole ou théophylline, les radicaux cités pouvant être une ou plusieurs fois substitués par un ou des groupes alkyle en $C_1$—$C_4$, alcoxy en $C_1$—$C_4$, phényle, phényl-alkyle($C_1$—$C_2$), hydroxy, amino ou hydroxyalkyle en $C_1$—$C_4$, ou par des groupes phényle ou amino substitués,

et sels pharmacologiquement acceptables de ceux-ci.

2. Composés selon la revendication 1, dans lesquels R est le groupe hydroxy et $R_2$ représente le radical

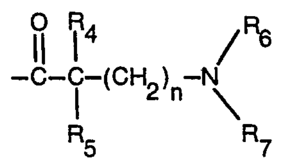

dans lequel $R_4$ et $R_5$ représentent des atomes d'hydrogène ou $R_4$ représente un atome d'hydrogène et $R_5$ représente un groupe alkyle en $C_1$—$C_4$, $R_6$ représente un groupe alkyle en $C_1$—$C_4$ et $R_7$ représente le groupe cyclohexyle, ou $R_6$ et $R_7$ représentent ensemble, conjointement avec l'atome d'azote auquel il sont liés, le radical pipéridino, morpholino, pipérazino ou pyrrolidino, et n est zéro, et sels pharmacologiquement acceptables de ceux-ci.

3. Médicaments, caractérisés par une teneur en un composé de formule I selon la revendication 1 et/ou en un sel pharmacologiquement acceptable.

4. Utilisation d'un composé de formule I et/ou d'un sel pharmacologiquement acceptable, pour la fabrication de médicaments.

5. Procédé pour la préparation d'un composé de formule I, dans lequel R, $R_1$ à $R_7$ ont les significations données dans la revendication 1, ainsi que de ses sels pharmaceutiquement acceptables, caractérisé en ce que

a) on fait réagir en présence d'une base un composé de formule II

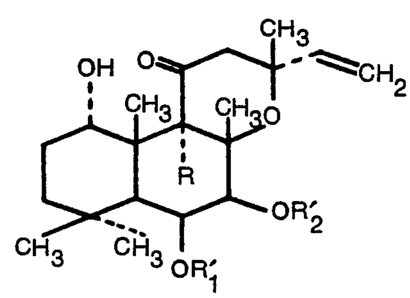

dans laquelle R représente un atome d'hydrogène ou le groupe hydroxy, et $R'_1$ et $R'_2$ représentent des atomes d'hydrogène, avec un composé de formule III

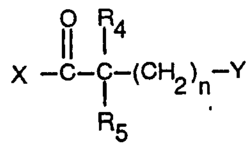

dans laquelle X et Y représentent des halogènes, et $R_4$, $R_5$ et n ont les significations données dans la revendication 1, et

b) on fait réagir le mélange obtenu de composés de formule IV

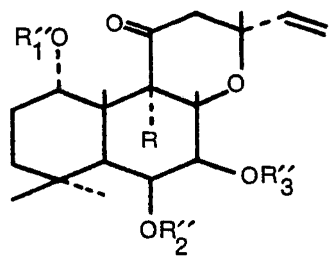

dans laquelle $R''_1$, $R''_2$ et $R''_3$ représentent des atomes d'hydrogène ou le radical

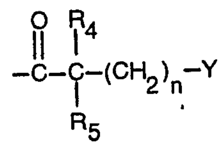

au moins l'un des substituants $R''_1$ à $R''_3$ représentant le radical mentionné, avec un composé de formule V

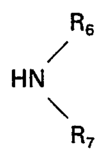

dans laquelle $R_6$ et $R_7$ ont les significations données dans la revendication 1, et on isole à la manière usuelle les composés de formule I à partir du mélange obtenu, et

c) éventuellement on fait réagir un composé de formule I, dans lequel R représente un atome d'hydrogène ou le groupe hydroxy, $R_1$ représente un atome d'hydrogène, $R_2$ représente un atome d'hydrogène et $R_3$ représente le radical

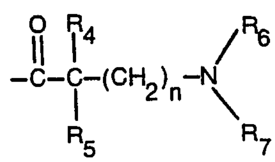

dans lequel $R_4$ à $R_7$ et n ont les significations données dans la revendication 1, avec un alcoolate alcalin en $C_1$—$C_4$, pour aboutir à un composé de formule I dans lequel $R_1$ représente un atome d'hydrogène, $R_2$ représente le radical

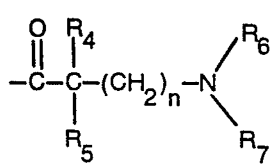

et $R_3$ représente un atome d'hydrogène.

6. Procédé selon la revendication 5, caractérisé en ce que l'on effectue la réaction d'un composé de formule I avec un composé de formule III en présence d'une base organique azotée, à une température dans la plage de 0°C à la température ambiante, et dans un hydrocarbure aromatique, un éther ou un hydrocarbure halogéné, et la réaction d'un composé de formule IV avec un composé de formule V pendant 1 à 6 heures à des températures dans la plage de la température ambiante à 150°C, avec ou sans solvants.

7. Procédé selon la revendication 5, caractérisé en ce que l'on traite en l'espace de 2 à 12 heures un composé de formule I par le méthylate de sodium, l'éthylate de sodium ou le tert-butylate de sodium, en présence d'hydrocarbures aromatiques ou d'éthers en tant que solvants.

**Revendications pour les Etats contractants: AT ES**

1. Procédé pour la préparation de composés de formule I

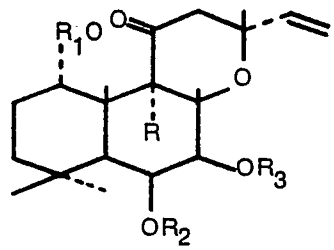

dans laquelle
R représente un atome d'hydrogène ou le groupe hydroxy;
$R_2$ représente le radical

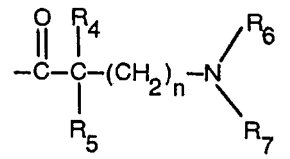

et
$R_1$ et $R_3$ représentent des atomes d'hydrogène;
$R_4$ et $R_5$ étant identiques ou différents, et représentant un atome d'hydrogène ou un groupe alkyle en $C_1$—$C_4$,
n étant zéro ou un nombre entier allant de 1 à 4,
$R_6$ représentant un atome d'hydrogène, et
$R_7$ représentant un atome d'hydrogène, un radical alkyle en $C_1$—$C_4$ substitué ou non substitué, cyclohexyle, phényle ou phényl-alkyle($C_1$—$C_2$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, un radical pyridine, quinoléine, amino éventuellement substitué, hydroxy, alcanoyle en $C_1$—$C_6$, alcénoyle en $C_2$—$C_6$, benzoyle ou phényl-alcanoyle-($C_1$—$C_6$), le reste phényle pouvant être une ou plusieurs fois substitué par un ou des atomes d'halogène ou groupes alkyle en $C_1$—$C_4$, alcoxy en $C_1$—$C_4$, nitro ou trifluorométhyle, un radical dialkyl($C_1$—$C_4$)-amino, carbamoyle, alcoxy($C_1$—$C_4$)-carbonyle, alcoxy($C_1$—$C_4$)-carbonyle, alcoxy($C_1$—$C_4$)-carbonyl-alkyle($C_1$—$C_4$), ou
$R_6$ et $R_7$ ont la même signification et représentent un radical alkyle en $C_1$—$C_4$ éventuellement substitué ou phényle ou phényl-alkyle($C_1$—$C_2$), les restes phényle pouvant être substitués comme indiqué plus haut, ou
$R_6$ représente un radical alkyle en $C_1$—$C_4$ et $R_7$ représente un radical alkyle en $C_1$—$C_4$ substitué ou cyclohexyle, phényl-alkyle($C_1$—$C_4$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, ou un radical dialkyl($C_1$—$C_4$)-aminoalkyle($C_1$—$C_4$), ou
$R_6$ et $R_7$ représentent ensemble, conjointement avec l'atome d'azote auquel il sont liés, le radical pipéridino, pyrrolidino, morpholino, pipérazino, thiomorpholino, imidazole ou théophylline, les radicaux cités pouvant être une ou plusieurs fois substitués par un ou des groupes alkyle en $C_1$—$C_4$, alcoxy en $C_1$—$C_4$, phényle, phényl-alkyle($C_1$—$C_2$), hydroxy, amino ou hydroxyalkyle en $C_1$—$C_4$, ou par des groupes phényle ou amino substitués,
ainsi que de leurs sels pharmacologiquement acceptables, caractérisé en ce que
a) on fait réagir en présence d'une base un composé de formule II

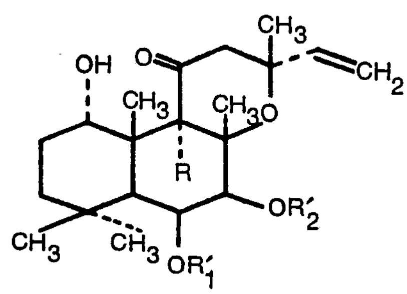

dans laquelle R représente un atome d'hydrogène ou le groupe hydroxy, et $R'_1$ et $R'_2$ représentent des atomes d'hydrogène, avec un composé de formule III

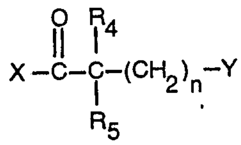

dans laquelle X et Y représentent des halogènes, et $R_4$, $R_5$ et n ont les significations données plus haut, et

b) on fait réagir le mélange obtenu de composés de formule IV

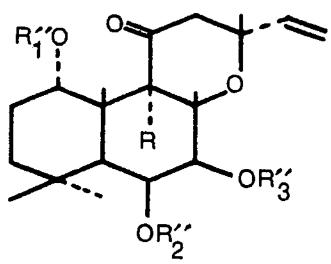

dans laquelle $R''_1$, $R''_2$ et $R''_3$ représentent des atomes d'hydrogène ou le radical

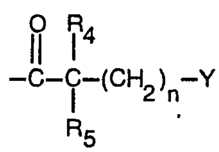

au moins l'un des substituants $R''_1$ à $R''_3$ représentant le radical mentionné, avec un composé de formule V

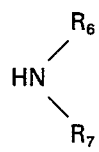

dans laquelle $R_6$ et $R_7$ ont les significations données plus haut, et on isole à la manière usuelle les composés de formule I à partir du mélange obtenu, et

c) éventuellement on fait réagir un composé de formule I, dans lequel R représente un atome d'hydrogène ou le groupe hydroxy, $R_1$ représente un atome d'hydrogène, $R_2$ représente un atome d'hydrogène et $R_3$ représente le radical

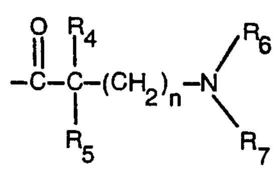

dans lequel $R_4$ à $R_7$ et n ont les significations données plus haut, avec un alcoolate en $C_1$—$C_4$, pour aboutir à un composé de formule I dans lequel $R_1$ représente un atome d'hydrogène, $R_2$ représente le radical

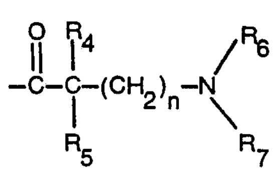

et $R_3$ représente un atome d'hydrogène.

2. Procédé selon la revendication 1, caractérisé en ce que l'on effectue la réaction d'un composé de formule I avec un composé de formule III en présence d'une base organique azotée, à une température dans la plage de 0°C à la température ambiante, et dans un hydrocarbure aromatique, un éther ou un hydrocarbure halogéné, et la réaction d'un composé de formule IV avec un composé de formule V pendant 1 à 6 heures à des températures dans la plage de la température ambiante à 150°C, avec ou sans solvants.

3. Procédé selon la revendication 1, caractérisé en ce que l'on traite en l'espace de 2 à 12 heures un composé de formule I par le méthylate de sodium, l'éthylate de sodium ou le tert-butylate de sodium, en présence d'hydrocarbures aromatiques ou d'éthers en tant que solvants.

4. Procédé selon les revendications 1 à 3, caractérisé en ce que R est le groupe hydroxy et $R_2$ représente le radical

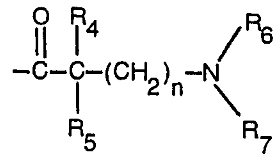

dans lequel $R_4$ et $R_5$ représentent des atomes d'hydrogène ou $R_4$ représente un atome d'hydrogène et $R_5$ représente un groupe alkyle en $C_1$—$C_4$, $R_6$ représente un groupe alkyle en $C_1$—$C_4$ et $R_7$ représente le groupe cyclohexyle, ou $R_6$ et $R_7$ représentent ensemble, conjointement avec l'atome d'azote auquel il sont liés, le radical pipéridino, morpholino, pipérazino ou pyrrolidino, et n est zéro.

5. Utilisation d'un composé de formule I et/ou d'un sel pharmacologiquement acceptable, pour la fabrication de médicaments.

**Revendications pour l'Etat contractant: GR**

1. Composés de formule I

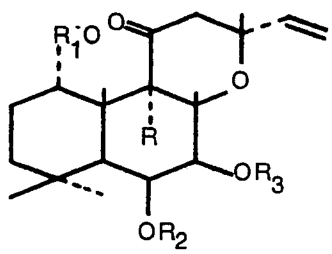

dans laquelle

R représente un atome d'hydrogène ou le groupe hydroxy;

$R_2$ représente le radical

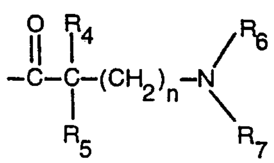

et

$R_1$ et $R_3$ représentent des atomes d'hydrogène;

$R_4$ et $R_5$ étant identiques ou différents, et représentant un atome d'hydrogène ou un groupe alkyle en $C_1$—$C_4$,

n étant zéro ou un nombre entier allant de 1 à 4,

$R_6$ représentant un atome d'hydrogène, et

$R_7$ représentant un atome d'hydrogène, un radical alkyle en $C_1$—$C_4$ substitué ou non substitué, cyclohexyle, phényle ou phényl-alkyle($C_1$—$C_2$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, un radical pyridine, quinoléine, amino éventuellement substitué, hydroxy, alcanoyle en $C_1$—$C_6$, alcénoyle en $C_2$—$C_6$, benzoyle ou phényl-alcanoyle-($C_1$—$C_6$), le reste phényle pouvant être une ou plusieurs fois substitué par un ou des atomes d'halogène ou groupes alkyle en $C_1$—$C_4$, alcoxy en $C_1$—$C_4$, nitro ou trifluorométhyle, un radical dialkyl($C_1$—$C_4$)-amino, carbamoyle, alcoxy($C_1$—$C_4$)-carbonyle, alcoxy($C_1$—$C_4$)-carbonyle, alcoxy($C_1$—$C_4$)-carbonyl-alkyle($C_1$—$C_4$), ou

$R_6$ et $R_7$ ont la même signification et représentent un radical alkyle en $C_1$—$C_4$ éventuellement substitué ou phényle ou phényl-alkyle($C_1$—$C_2$), les restes phényle pouvant être substitués comme indiqué plus haut, ou

$R_6$ représente un radical alkyle en $C_1$—$C_4$ et $R_7$ représente un radical alkyle en $C_1$—$C_4$ substitué ou cyclohexyle, phényl-alkyle($C_1$—$C_4$), le reste phényle pouvant être une ou plusieurs fois substitué par un ou des atomes d'halogène ou groupes alkyle en $C_1$—$C_4$, alcoxy en $C_1$—$C_4$, nitro et/ou trifluorométhyle, ou un radical dialkyl($C_1$—$C_4$)-aminoalkyle($C_1$—$C_4$), ou

$R_6$ et $R_7$ représentent ensemble, conjointement avec l'atome d'azote auquel il sont liés, le radical pipéridino, pyrrolidino, morpholino, pipérazino, thiomorpholino, imidazole ou théophylline, les radicaux cités pouvant être une ou plusieurs fois substitués par un ou des groupes alkyle en $C_1$—$C_4$, alcoxy en $C_1$—$C_4$, phényle, phényl-alkyle($C_1$—$C_2$), hydroxy, amino ou hydroxyalkyle en $C_1$—$C_4$, ou par des groupes phényle ou amino substitués,

et sels pharmacologiquement acceptables de ceux-ci.

2. Composés selon la revendication 1, dans lesquels R est le groupe hydroxy et $R_2$ représente le radical

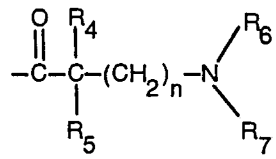

dans lequel $R_4$ et $R_5$ représentent des atomes d'hydrogène ou $R_4$ représente un atome d'hydrogène et $R_5$ représente un groupe alkyle en $C_1$—$C_4$, $R_6$ représente un groupe alkyle en $C_1$—$C_4$ et $R_7$ représente le groupe cyclohexyle, ou $R_6$ et $R_7$ représentent ensemble, conjointement avec l'atome d'azote auquel il sont liés, le radical pipéridino, morpholino, pipérazino ou pyrrolidino, et n est zéro, et sels d'addition avec des acides pharmacologiquement acceptables de ceux-ci.

3. Utilisation d'un composé de formule I et/ou d'un sel pharmacologiquement acceptable, pour la fabrication de médicaments.

4. Procédé pour la préparation d'un composé de formule I, dans lequel R, $R_1$ à $R_7$ ont les significations données dans la revendication 1, ainsi que de ses sels pharmaceutiquement acceptables, caractérisé en ce que

a) on fait réagir en présence d'une base un composé de formule II

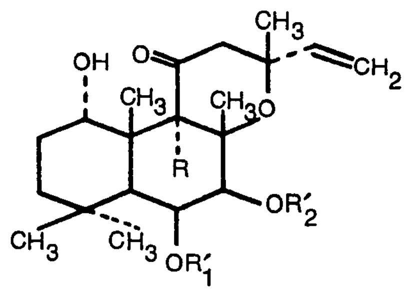

dans laquelle R représente un atome d'hydrogène ou le groupe hydroxy, et $R'_1$ et $R'_2$ représentent des atomes d'hydrogène, avec un composé de formule III

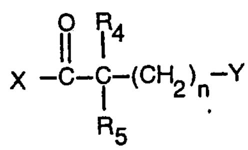

dans laquelle X et Y représentent des halogènes, et $R_4$, $R_5$ et n ont les significations données dans la revendication 1, et

b) on fait réagir le mélange obtenu de composés de formule IV

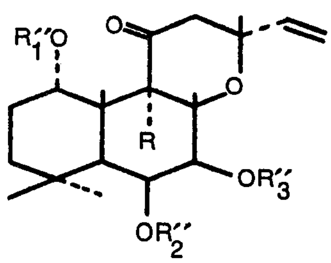

dans laquelle $R''_1$, $R''_2$ et $R''_3$ représentent des atomes d'hydrogène ou le radical

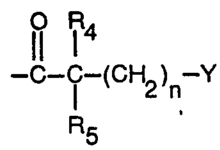

au moins l'un des substituants $R''_1$ à $R''_3$ représentant le radical mentionné, avec un composé de formule V

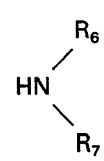

dans laquelle $R_6$ et $R_7$ ont les significations données dans la revendication 1, et on isole à la manière usuelle les composés de formule I à partir du mélange obtenu, et

c) éventuellement on fait réagir un composé de formule I, dans lequel R représente un atome d'hydrogène ou le groupe hydroxy, $R_1$ représente un atome d'hydrogène, $R_2$ représente un atome d'hydrogène et $R_3$ représente le radical

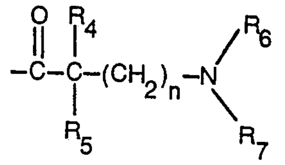

dans lequel $R_4$ à $R_7$ et n ont les significations données dans la revendication 1, avec un alcoolate alcalin en $C_1$—$C_4$, pour aboutir à un composé de formule I dans lequel $R_1$ représente un atome d'hydrogène, $R_2$ représente le radical

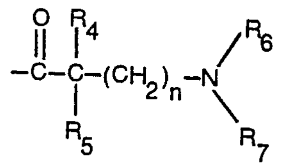

et $R_3$ représente un atome d'hydrogène.

5. Procédé selon la revendication 4, caractérisé en ce que l'on effectue la réaction d'un composé de formule I avec un composé de formule III en présence d'une base organique azotée, à une température dans la plage de 0°C à la température ambiante, et dans une hydrocarbure aromatique, un éther ou un hydrocarbure halogéné, et la réaction d'un composé de formule IV avec un composé de formule V pendant 1 à 6 heures à des températures dans la plage de la température ambiante à 150°C, avec ou sans solvants.

6. Procédé selon la revendication 4, caractérisé en ce que l'on traite en l'espace de 2 à 12 heures un composé de formule I par le méthylate de sodium, l'éthylate de sodium ou le tert-butylate de sodium, en présence d'hydrocarbures aromatiques ou d'éthers en tant que solvants.

**Claims for the Contracting States: BE CH DE FR GB IT LI LU NL SE**

1. A compound of the formula I

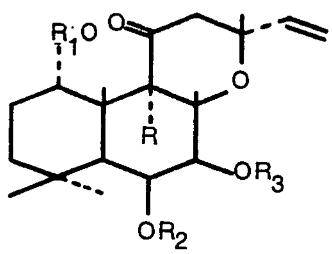

where

R denotes hydrogen or hydroxyl;

$R_2$ denotes the radical

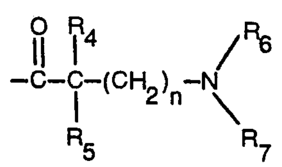

and $R_1$ and $R_3$ denote hydrogen, where

$R_4$ and $R_5$ are identical or different and represent hydrogen or $(C_1$—$C_4)$alkyl,

n stands for 0 or an integer from 1 to 4,

$R_6$ denotes hydrogen and

$R_7$ represents hydrogen, unsubstituted or substituted $(C_1$—$C_4)$alkyl, cyclohexyl, phenyl or phenyl$(C_1$—$C_2)$alkyl, where the phenyl radical may be singly or multiply substituted by halogen, $(C_1$—$C_4)$alkyl, $(C_1$—$C_4)$alkoxy, nitro and/or trifluoromethyl, pyridine, quinoline, optionally substituted amino, hydroxyl, $(C_1$—$C_6)$alkanoyl, $(C_2$—$C_6)$alkenoyl, benzoyl or phenyl$(C_1$—$C_6)$alkanoyl where the phenyl radical may be singly or multiply substituted by halogen, $(C_1$—$C_4)$alkyl, $(C_1$—$C_4)$alkoxy, nitro or trifluoromethyl, di$(C_1$—$C_4)$alkylamino, carbamyl, $(C_1$—$C_4)$alkoxycarbonyl, $(C_1$—$C_4)$alkoxycarbonyl$(C_1$—$C_4)$alkyl, or

$R_6$ and $R_7$ have the same meaning and represent optionally substituted $(C_1$—$C_4)$alkyl or phenyl or phenyl$(C_1$—$C_2)$alkyl, where the phenyl radicals may be substituted as specified above, or

$R_6$ denotes $(C_1$—$C_4)$alkyl and $R_7$ represents substituted $(C_1$—$C_4)$alkyl or cyclohexyl, phenyl-

$(C_1—C_4)$alkyl, where the phenyl radical may be singly or multiply substituted by halogen, $(C_1—C_4)$alkyl, $(C_1—C_4)$alkoxy, nitro and/or trifluoromethyl, or di$(C_1—C_4)$alkylamino$(C_1—C_4)$alkyl, or

$R_6$ and $R_7$ together with the N atom to which they are attached represent the piperidinyl, pyrrolidinyl, morpholinyl, piperazinyl, thiomorpholinyl, imidazolyl or theophyllinyl radical, where the said radicals may be singly or multiply substituted by $(C_1—C_4)$alkyl, $(C_1—C_4)$alkoxy, phenyl$(C_1—C_2)$alkyl, hydroxyl, amino- or hydroxyl$(C_1—C_4)$alkyl or substituted phenyl or amino groups, and also pharmacologically acceptable salts thereof.

2. A compound as claimed in claim 1, where R is hydroxyl and $R_2$ denotes the radical

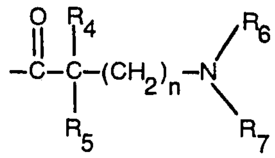

where $R_4$ and $R_5$ represent hydrogen or $R_4$ represents hydrogen and $R_5$ represents $(C_1—C_4)$alkyl, $R_6$ represents $(C_1—C_4)$alkyl and $R_7$ represents cyclohexyl, or $R_6$ and $R_7$ together with the N atom to which they are attached represent the piperidinyl, morpholinyl, piperazinyl or pyrrolidinyl radical and n is 0, and also pharmacologically acceptable salts thereof.

3. A drug which contains a compound of the formula I as claimed in claim 1 and/or a pharmaceutically acceptable salt.

4. The use of a compound of the formula I and/or a pharmacologically acceptable salt for the preparation of drugs.

5. A process for the preparation of a compound of the formula I where R, $R_1$ to $R_7$ and n have the meaning stated in claim 1 and also pharmacologically acceptable salts thereof which comprises

a) reacting a compound of the formula II

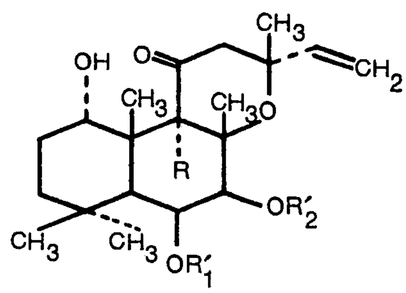

where R represents hydrogen or hydroxyl and $R'_1$ and $R'_2$ are hydrogen, with a compound of the formula III

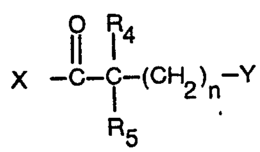

where X and Y denote halogen and $R_4$, $R_5$ and n have the meanings stated in claim 1, in the presence of a base, and

b) reacting the mixture of compounds obtained of the formula IV

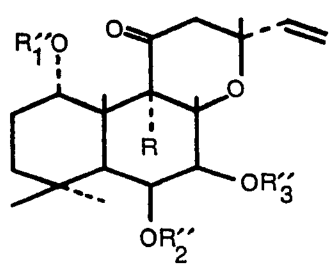

where $R''_1$, $R''_2$ and $R''_3$ denote hydrogen or the radical

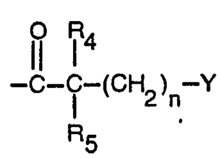

where at least one of the substituents $R''_1$ to $R''_3$ represents the said radical, with a compound of the formula V

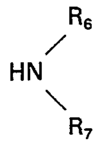

where $R_6$ and $R_7$ have the meanings stated in claim 1, and isolating the compounds of the formula I from the mixture obtained in the usual manner, and

c) optionally reacting a compound of the formula I, where R denotes hydrogen or hydroxyl, $R_1$ represents hydrogen, $R_2$ represents hydrogen and $R_3$ represents the radical

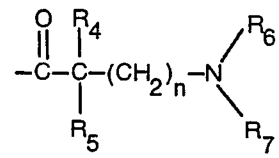

where $R_4$ to $R_7$ and n have the meanings stated in claim 1, with an alkali($C_1$—$C_4$)alkoxide to form a compound of the formula I, where $R_1$ denotes hydrogen, $R_2$ denotes the radical

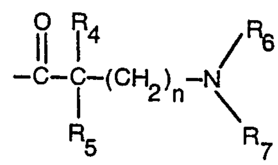

and $R_3$ denotes hydrogen.

6. The process as claimed in claim 5, wherein the reaction of a compound of the formula I with a compound of the formula III is carried out in the presence of an organic nitrogen base at a temperature from 0°C to room temperature and in an aromatic hydrocarbon, an ether or in a halogenated hydrocarbon, and the reaction of a compound of the formula IV with a compound of the formula V is carried out for 1 to 6 hours at temperatures from room temperature up to 150°C with or without solvents.

7. The process as claimed in claim 5, wherein a compound of the formula I is treated with sodium methoxide, sodium ethoxide or sodium tert-butoxide in the presence of aromatic hydrocarbons or ethers as solvents over a period of 2 to 12 hours.

**Claims for the Contracting States: AT ES**

1. A process for the preparation of a compound of the formula I

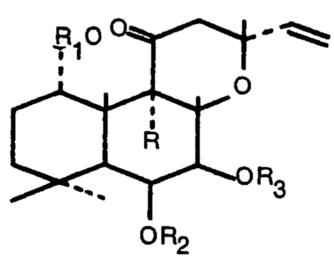

where

R denotes hydrogen or hydroxyl;

$R_2$ denotes the radical

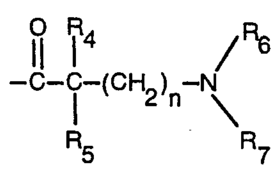

and $R_1$ and $R_3$ denote hydrogen, where

$R_4$ and $R_5$ are identical or different and represent hydrogen or ($C_1$—$C_4$)alkyl,

n stands for 0 or an integer from 1 to 4,

$R_6$ denotes hydrogen and

$R_7$ represents hydrogen, unsubstituted or substituted $(C_1—C_4)$alkyl, cyclohexyl, phenyl or phenyl$(C_1—C_2)$alkyl, where the phenyl radical may be singly or multiply substituted by halogen, $(C_1—C_4)$alkyl, $(C_1—C_4)$alkoxy, nitro and/or trifluoromethyl, pyridine, quinoline, optionally substituted amino, hydroxyl, $(C_1—C_6)$alkanoyl, $(C_2—C_6)$alkenoyl, benzoyl or phenyl$(C_1—C_6)$alkanoyl where the phenyl radical may be singly or multiply substituted by halogen, $(C_1—C_4)$alkyl, $(C_1—C_4)$alkoxy, nitro or trifluoromethyl, di$(C_1—C_4)$alkylamino, carbamoyl, $(C_1—C_4)$alkoxycarbonyl, $(C_1—C_4)$alkoxycarbonyl$(C_1—C_4)$alkyl, or

$R_6$ and $R_7$ have the same meaning and represent optionally substituted $(C_1—C_4)$alkyl or phenyl or phenyl$(C_1—C_2)$alkyl, where the phenyl radicals may be substituted as specified above, or

$R_6$ denotes $(C_1—C_4)$alkyl and $R_7$ represents substituted $(C_1—C_4)$alkyl or cyclohexyl, phenyl-$(C_1—C_4)$alkyl, where the phenyl radical may be singly or multiply substituted by halogen, $(C_1—C_4)$alkyl, $(C_1—C_4)$alkoxy, nitro and/or trifluoromethyl, or di$(C_1—C_4)$alkylamino$(C_1—C_4)$alkyl, or

$R_6$ and $R_7$ together with the N atom to which they are attached represent the piperidinyl, pyrrolidinyl, morpholinyl, piperazinyl, thiomorpholinyl, imidazolyl or theophyllinyl radical, where the said radicals may be singly or multiply substituted by $(C_1—C_4)$alkyl, $(C_1—C_4)$alkoxy, phenyl$(C_1—C_2)$alkyl, hydroxyl, amino- or hydroxyl$(C_1—C_4)$alkyl or substituted phenyl or amino groups, and also pharmacologically acceptable salts thereof,

which comprises

a) reacting a compound of the formula II

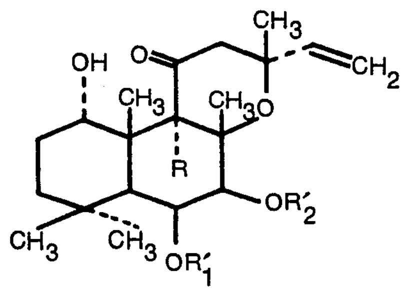

where R represents hydrogen or hydroxyl and $R'_1$ and $R'_2$ are hydrogen, with a compound of the formula III

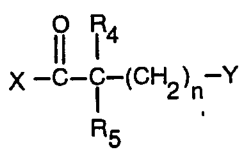

where X and Y denote halogen and $R_4$, $R_5$ and n have the abovementioned meanings, in the presence of a base, and

b) reacting the mixture of compounds obtained of the formula IV

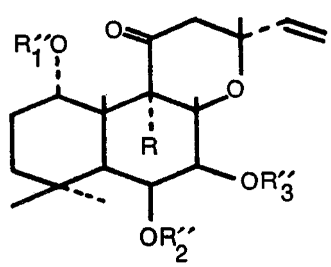

where $R''_1$, $R''_2$ and $R''_3$ denote hydrogen or the radical

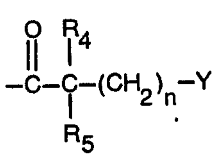

where at least one of the substituents $R''_1$ to $R''_3$ represents the said radical with a compound of the formula V

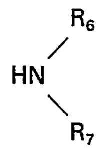

where $R_6$ and $R_7$ have the abovementioned meanings, and isolating the compounds of the formula I from the mixture obtained in the usual manner, and

c) optionally reacting a compound of the formula I, where R denotes hydrogen or hydroxyl, $R_1$ represents hydrogen, $R_2$ represents hydrogen and $R_3$ represents the radical

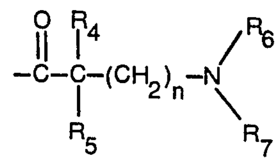

where $R_4$ to $R_7$ and n have the abovementioned meanings, with an alkali($C_1$—$C_4$)alkoxide to form a compound of the formula I, where $R_1$ denotes hydrogen, $R_2$ denotes the radical

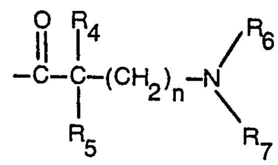

and $R_3$ denotes hydrogen.

2. The process as claimed in claim 1, wherein the reaction of a compound of the formula I with a compound of the formula III is carried out in the presence of an organic nitrogen base at a temperature from 0°C to room temperature and in an aromatic hydrocarbon, an ether or in a halogenated hydrocarbon, and the reaction of a compound of the formula IV with a compound of the formula V is carried out for 1 to 6 hours at temperatures from room temperature up to 150°C with or without solvents.

3. The process as claimed in claim 1, wherein a compound of the formula I is treated with sodium methoxide, sodium ethoxide or sodium tert-butoxide in the presence of aromatic hydrocarbons or ethers as solvents over a period of 2 to 12 hours.

4. The process as claimed in claims 1 to 3, wherein R is hydroxyl and $R_2$ denotes the radical

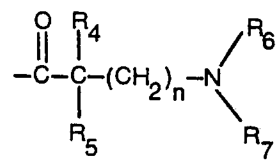

where $R_4$ and $R_5$ represent hydrogen or $R_4$ represents hydrogen and $R_5$ represents ($C_1$—$C_4$)alkyl, $R_6$ represents ($C_1$—$C_4$)alkyl and $R_7$ represents cyclohexyl, or $R_6$ and $R_7$ together with the N atom to which they are attached represent the piperidinyl, morpholinyl, piperazinyl, or pyrrolidinyl radical and n is 0.

5. The use of a compound of the formula I and/or a pharmacologically acceptable salt for the preparation of drugs.

**Claims for the Contracting State: GR**

1. A compound of the formula I

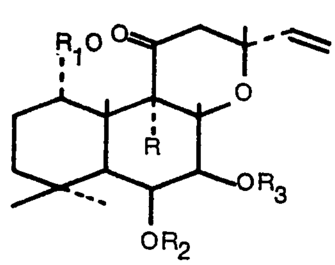

where

R denotes hydrogen or hydroxyl;

$R_2$ denotes the radical

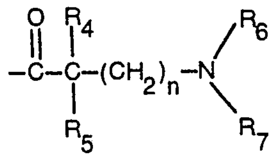

and $R_1$ and $R_3$ denote hydrogen, where

$R_4$ and $R_5$ are identical or different and represent hydrogen or $(C_1—C_4)$alkyl,

n stands for 0 or an integer from 1 to 4,

$R_6$ denotes hydrogen and

$R_7$ represents hydrogen, unsubstituted or substituted $(C_1—C_4)$alkyl, cyclohexyl, phenyl or phenyl$(C_1—C_2)$alkyl, where the phenyl radical may be singly or multiply substituted by halogen, $(C_1—C_4)$alkyl, $(C_1—C_4)$alkoxy, nitro and/or trifluoromethyl, pyridine, quinoline, optionally substituted amino, hydroxyl, $(C_1—C_6)$alkanoyl, $(C_2—C_6)$alkenoyl, benzoyl or phenyl$(C_1—C_6)$alkanoyl where the phenyl radical may be singly or multiply substituted by halogen, $(C_1—C_4)$alkyl, $(C_1—C_4)$alkoxy, nitro or trifluoromethyl, di$(C_1—C_4)$alkylamino, carbamoyl, $(C_1—C_4)$alkoxycarbonyl, $(C_1—C_4)$alkoxycarbonyl$(C_1—C_4)$alkyl, or

$R_6$ and $R_7$ have the same meaning and represent optionally substituted $(C_1—C_4)$alkyl or phenyl or phenyl$(C_1—C_2)$alkyl, where the phenyl radicals may be substituted as specified above, or

$R_6$ denotes $(C_1—C_4)$alkyl and $R_7$ represents substituted $(C_1—C_4)$alkyl or cyclohexyl, phenyl-$(C_1—C_4)$alkyl, where the phenyl radical may be singly or multiply substituted by halogen, $(C_1—C_4)$alkyl, $(C_1—C_4)$alkoxy, nitro and/or trifluoromethyl, or di$(C_1—C_4)$alkylamino$(C_1—C_4)$alkyl, or

$R_6$ and $R_7$ together with the N atom to which they are attached represent the piperidinyl, pyrrolidinyl, morpholinyl, piperazinyl, thiomorpholinyl, imidazolyl or theophyllinyl radical, where the said radicals may be singly or multiply substituted by $(C_1—C_4)$alkyl, $(C_1—C_4)$alkoxy, phenyl, phenyl$(C_1—C_2)$alkyl, hydroxyl, amino- or hydroxyl$(C_1—C_4)$alkyl or substituted phenyl or amino groups, and also pharmacologically acceptable salts thereof.

2. A compound as claimed in claim 1, where R is hydroxyl and $R_2$ denotes the radical

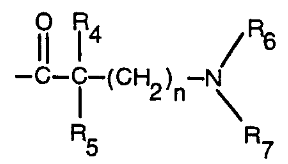

where $R_4$ and $R_5$ represent hydrogen or $R_4$ represents hydrogen and $R_5$ represents $(C_1—C_4)$alkyl, $R_6$ represents $(C_1—C_4)$alkyl and $R_7$ represents cyclohexyl, or $R_6$ and $R_7$ together with the N atom to which they are attached represent the piperidinyl, morpholinyl, piperazinyl or pyrrolidinyl radical and n is 0, and also pharmacologically acceptable acid addition salts thereof.

3. The use of a compound of the formula I and/or a pharmacologically acceptable salt for the preparation of drugs.

4. A process for the preparation of a compound of the formula I where R, $R_1$ to $R_7$ and n have the meaning stated in claim 1 and also pharmacologically acceptable salts thereof which comprises

a) reacting a compound of the formula II

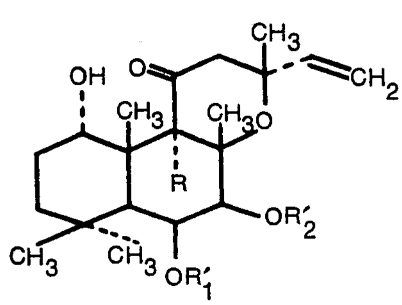

where R represents hydrogen or hydroxyl and $R'_1$ and $R'_2$ are hydrogen, with a compound of the formula III

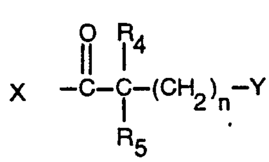

where X and Y denote halogen and $R_4$, $R_5$ and n have the meanings stated in claim 1, in the presence of a base, and

b) reacting the mixture of compounds obtained of the formula IV

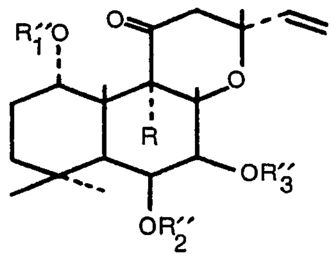

where $R''_1$, $R''_2$ and $R''_3$ denote hydrogen or the radical

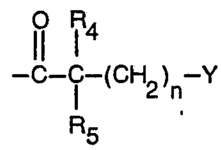

where at least one of the substituents $R''_1$ to $R''_3$ represents the said radical, with a compound of the formula V

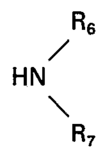

where $R_6$ and $R_7$ have the meanings stated in claim 1, and isolating the compounds of the formula I from the mixture obtained in the usual manner, and

c) optionally reacting a compound of the formula I, where R denotes hydrogen or hydroxyl, $R_1$ represents hydrogen, $R_2$ represents hydrogen and $R_3$ represents the radical

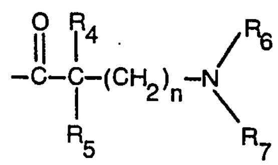

where $R_4$ to $R_7$ and n have the meanings stated in claim 1, with an alkali($C_1$—$C_4$)alkoxide to form a compound of the formula I, where $R_1$ denotes hydrogen, $R_2$ denotes the radical

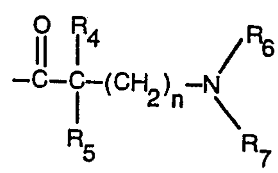

and $R_3$ denotes hydrogen.

5. The process as claimed in claim 4, wherein the reaction of a compound of the formula I with a compound of the formula III is carried out in the presence of an organic nitrogen base at a temperature from 0°C to room temperature and in an aromatic hydrocarbon, an ether or in a halogenated hydrocarbon, and the reaction of a compound of the formula IV with a compound of the formula V is carried out for 1 to 6 hours at temperatures from room temperature up to 150°C with or without solvents.

6. The process as claimed in claim 4, wherein a compound of the formula I is treated with sodium methoxide, sodium ethoxide or sodium tert-butoxide in the presence of aromatic hydrocarbons or ethers as solvents over a period of 2 to 12 hours.